# EUROPEAN PATENT APPLICATION

(11) **EP 2 380 992 A1**
(43) Date of publication of application: **26.10.2011**
(21) Application number: 10004228.2
(22) Date of filing: 21.04.2010
(51) Int. Cl.: C12Q 1/68

(54) **Methods for predicting an antibody response to interfer on therapy in multiple sclerosis patients**

(71) Applicant: Max-Planck-Gesellschaft zur Förderung der Wissenschaften e.V., 80539 München (DE)
(72) Inventor: Weber, Frank, 85521 Ottobrunn (DE); Müller-Myhsok, Bertram, 81241 München (DE); Holsboer, Florian, 80805 München (DE); Hemmer, Bernhard, 81245 München (DE)
(74) Representative: Vossius & Partner

(57) **Abstract**

The present invention relates to a method of diagnosing a predisposition of a multiple sclerosis patient to become less susceptible or resistant to treatment with interferon β, said method comprising determining in a sample obtained from said patient the presence of one or more SNP(s) selected from the group consisting of SNPs defined by the sequences of SEQ ID NOs 1, 2, 3, 4, 5, 6, 7, 8, 9, 10, 11, 12, 13, 14, 15, 16 and 17, wherein the presence of one or more of said SNP(s) in said sample is indicative for said predisposition.

SEQUENCE LISTING

<110> Max-Planck-Gesellschaft zur Förderung der Wissenschaften
e.V.

<120> Methods for predicting an antibody response to interferon therapy
in multiple sclerosis patients

<130> P2461 EP

<160> 113

<170> PatentIn version 3.3

<210> 1
<211> 400
<212> DNA
<213> Homo sapiens

<220>
<221> variation
<222> 200
<223> /replace="t"

<400> 1
tttctcaaaa aacatcaaca aaaataaaaa aaataaaatt tgttacattc tacaaacttg 60

gtctaaactt ggtctaattc ctttgcattt agggagccgt gggtatgaac acatgttttg 120

ggaatgctgg ggtgaagagg tgcgaaagct ctgtctactt tgatctccag aaactaagtc 180

ataaatgtta gaagaggggg tgaaaaggct ttgtaatgac aaggccttaa aaaatgtgaa 240

agctatcttt attttcataa ctaatgatgg taaccattaa ccatcatgtg cctatcatcc 300

ttttaatcaa atcccagagg caactataaa gagaattctt gtcctagact agctagattt 360

ccaaaggcag ggtctgtata agcccctccc tataggcagc 400

<210> 2
<211> 401
<212> DNA
<213> Homo sapiens

<220>
<221> variation
<222> 201
<223> /replace="c"

<400> 2
acacaggttc cactatctga ccctcgttcc catggccgag gacacctgtg acctccaggg 60

ggagcactgg ggcctgcacc agcctctcct caggcactgg ggtatggagc gccctccctc 120

tgccctcacg gccttggcac cacctttatt tcctgggccc atcgcccctc agcacctgcc 180

ttcctcaatc ccatgtttta tggtcacttt atccaaattt caccatctca tggtttcgaa 240

tacccaacac ctcccacatc caaggccagc ccctgctctc tgtaccttat aactctgtct 300

tcccttggtg ccccagaggt ccatgaacta atccaggtgc ctgagaccat ggagatgctg 360

gtctgtgccc tcggcctgct ggtgggcctg gcgggggtcc t 401

<210> 3
<211> 401

<212>  DNA
<213>  Homo sapiens

<220>
<221>  variation
<222>  201
<223>  /replace="g"

<400>  3
ctctcaggct tgctctgccc caggtttctg aacgtggaat acatttctcc agggaaactc      60

agtattatga gatttgggag gtggaagtta ggccacagcc atctcaggga caggtttcac     120

agacatgagt tttggcagca gccttgtgtt ctaaagacat ttactcctag gggctctaga     180

ggatctgcaa catcagcaga agcttcctgt gggttcctga tcttttaaaa ttagggttct     240

gcagtgactt ctgctcctcc agacccccta acagttttaa gggctaattc cctgtaatat     300

attcagttct gcttagactg attacaggga ttcctatttc ttgactgaat tctcatggct     360

atagtggctc gtcaccattt gacatcacca agaagtcctc a                         401

<210>  4
<211>  401
<212>  DNA
<213>  Homo sapiens

<220>
<221>  variation
<222>  201
<223>  /replace="c"

<400>  4
tctggtatgt tacaaaagga ttacattttc cctccacctg catgggggag tatttctcca      60

atattcattg tgatgacctc gtgggacccc tagagataaa actcacaaaa gtgtgaaggc     120

cctctatgac tgtctcccct ggagttctta actctcaaat ttgtccacag tgaacctcca     180

gcatctcatt aataatacag tttttcttaa cagcactagt tcccatgaaa tttctgctcc     240

agtaagttgt gatttctgta tgcgtttgtc tatctctcca aactgtggga aagcaaattg     300

ccctgcaatt tcacttaaat tctctgatgg acctaagaag aattgttgat ttttcaatat      360

gttcagttct ttatttgttg ttaggatttc taagctactt a                         401

<210>  5
<211>  401
<212>  DNA
<213>  Homo sapiens

<220>
<221>  variation
<222>  201
<223>  /replace="t"

<400>  5
tgcatttctg gaataaactc cacttggtca tgatgtagta gccatctttt atatcaatga      60

attcagttgg ctattatttt cttaagaact tggctcataa gaattattgg tccataaatt     120

cctatttgtt gtagagttct tttcagatct tgaagtaaat tttacacaga tctcatcaaa     180

cctaactgca agtgttttcc cctttttcaa atgtttgaag tttgcataaa gttagtgttg     240

tttgttcctt aaatatttta aaatatttac tgaagctaaa tgaacctggg gttttgtctt 300

gggaaagctt tatagaattg gctttctcta tgagatgtgg aactcttcat actttatgtt 360

atttgttagc ttcagtacat tgcacatttt gagtaattta t 401

<210> 6
<211> 400
<212> DNA
<213> Homo sapiens

<220>
<221> variation
<222> 200
<223> /replace="c"

<400> 6
attctctatt aggatcaagc tgagaatcct tcagatctct aaattctgtt tcccttttaa 60

ttataaattc catctttaaa tcatttcttt cttctcatat tttcctttat acacttaaag 120

gaagccatgc aatattctga gtattttact gattagatat ttcttctgcc agacatccaa 180

attcatcacc ctctacttct gcctttcatg aagtcctagg acatgaccac aattcaacca 240

agttctttgc cactttataa caaggatggc cttttctcta attttcaata ccttgttctt 300

tatttccatc tgagacttca tcagaattgc ctttaccatt catatttcta ccaacattct 360

gatcacatct acttagagat tatctaagta atctctagga 400

<210> 7
<211> 401
<212> DNA
<213> Homo sapiens

<220>
<221> variation
<222> 201
<223> /replace="g"

<400> 7
catgaacttt ccaactcact tcctgaaaaa ctggccacat agctacaatt atttgtcacc 60

cccttggtaa ctccatggga atactttgct gaaagcctgg catggtcccc gtgcttccaa 120

cgcacaactt cctgatattc tgtgcttgcc aattcgtaag taagggtgaa aaagttgaaa 180

ctccttgtct tttgtatgaa ccagtagttg gaaaagtgtg tcacacctca tgcaggaagt 240

tatagaccta agtagggttt attgaagcgt cctataaatc tccctagaat atagaaattc 300

acttcaaggg aggagaggaa taaccaagtg atctgccatt ttcattgttg gaaaaaaaat 360

atcagtattc atttaaaaga ggtctacttt atgagtcaag c 401

<210> 8
<211> 401
<212> DNA
<213> Homo sapiens

<220>
<221> variation
<222> 201

<223>  /replace="a"

<400>  8
tgctgggatt acagtcgtga gccaccgcgc ctggcctcct catattattt tttattgtgc      60

agtttattca agtgagttat ttaaaacaac tagttcacac acataggagt tgttgctgat     120

aaagaattgg tggaaatgat attaaataac aattgttttt ataagtttcc tctgcttcat     180

taattttatg attgtgagag gccatgtgac tggatatcag catacaggac tttgtatcag     240

aatatgagct tggcagtcat aagagattac ttacaattct tcacaattct gtattttcat     300

ttgtaaaact ccataaagtt ttttataaga ataaattgta gcaccttatt catactggaa     360

ttcaacagtt cttagttcaa gtctcccttg agaaagcttc a                         401

<210>  9
<211>  401
<212>  DNA
<213>  Homo sapiens

<220>
<221>  variation
<222>  201
<223>  /replace="t"

<400>  9
ccagcctgaa gcctcaaaaa agaaaaaaaa acttgattga gaaaaattaa agtacaacat      60

ttggctctga acttcctggc agccaagaca aaatgataca aagaagatct ctgatctttg     120

tcaagacacc ctgctttatg cctggtcccc aaagtgagca gaagtgaggc accttgccct     180

ctaaagcaat tcctttcttt ctttctttct tttttttttt catttgaagg atttcttttt     240

taaactttta ttttaagtta aggggtacat gtgtaggttt gtttcatggg taaatgtgtg     300

tcacaggatg ggggtttgtt tacggattat ttcatcatcc aggtattaag cctagtatcc     360

attagttatt tttcgtgatc gtttccctcc ccccaacctc c                         401

<210>  10
<211>  401
<212>  DNA
<213>  Homo sapiens

<220>
<221>  variation
<222>  201
<223>  /replace="g"

<400>  10
taaatctctt taggtgactc aaaactacag gtaggcttaa caacccctag ggccttgatg      60

atagcaaaaa acttttaaaa tcgttcttgt ggataatatt ctggggagca gaccatgaac     120

gagtgaaagg ataatgggtg cactgacagc cctatgaaga ttagacccct caatagacaa     180

gggcactact cccgctggtc atatgagtgt ctatgttagc actcaagctg cccaaccaga     240

gagggaagag gattgtagga tttcctagga atggcgaagc ctaatgggat ggaatagagg     300

agtttgctga aacattagtg ccagtgaaaa tcaatgatac gggctagtta gggaaggaag     360

ccagatggac tggaagaaaa aaaaaatcat gaaactgtaa g                         401

<210> 11
<211> 401
<212> DNA
<213> Homo sapiens

<220>
<221> variation
<222> 201
<223> /replace="a"

<400> 11
ctagtcaacc tggcactgag ctgggacctg tgtacacttt gcctgtaact cttacagtat 60

cctgagaggt agatcatatc aagctcatat tatttataaa gaactgagac tcagagatga 120

taagtaaatt atactaaatc atataattag tgaatgatag agaacagata cgaaaccaag 180

attttctctt ctaaagccca gttccagggt caagatagta cagagatgag tttgttcatt 240

tgtttgtttc tttgttttgt gtgcttctta gcagttgcgg ttattgaata agtcggccca 300

aggggctact ttagtcaaaa actcaaaagt atttcaaaat tctgaagaat gaccacataa 360

atctcccctt gatggcacat aaattttgct ttgcaagtca t 401

<210> 12
<211> 401
<212> DNA
<213> Homo sapiens

<220>
<221> variation
<222> 201
<223> /replace="a"

<400> 12
tgatacaagg ccaatggagt cactaacgac tcactgggga ccagggatgt cttcctggag 60

gaggtggaat cggagcaggg cttcggagag tggacgggat ttggggtaga ccagggtttc 120

acaaccttga cattgacatc tggggatcat tctctggtga ggggccgttc cgtgcactgc 180

agggtgctga gcaacatccc gggcctccac gcatttgatg ccacttggca cccccagacc 240

ccgaacagtt gtgacaatca aaagcatttc gagaaattgc caaatgttcc ctgggaacaa 300

aatttacccc agttgatttt tttttttttt tttttttttt gagatggagt ctcattcttg 360

cccgggctgg agtgcggtcg tgtgatctcg gctcactgca a 401

<210> 13
<211> 401
<212> DNA
<213> Homo sapiens

<220>
<221> variation
<222> 201
<223> /replace="g"

<400> 13
gcatacattt atgaaatggc actgcttcct acatgcaaaa gtagaaacag ccatgttatg 60

caaaattatc tatgacatcc aacccactgg ggctcatata caacttatgg tttcattatg 120

cagaattata atgggtctta atattctatt actccttggc tctaatacct tttcatcaac 180

gtaaggacct taagattagg tctttgctta gattggctag cattgtcacc ctggaaactc 240

tagtgtccta tggaattaca caaattgata atctaaagat agcaagcatg tcagaaattt 300

cagaagtagt tataatgtat ttgttaataa ttcatggtta gcaacaggga tatgaaatta 360

caaatgactg aacttcagtt tcctgatcat atatgattac t 401

<210> 14
<211> 401
<212> DNA
<213> Homo sapiens

<220>
<221> variation
<222> 201
<223> /replace="a"

<400> 14
tactctcagt cacactcact ctgctctagc cctaccaggc ttcctccagg cctcttacct 60

gacatatttt ctcatgtcac atgagctttt ccaatggtct ttcttctgtg cagaatgctc 120

ttccaacccc ctttacctag ttaactccta ttcatcctct gaattgctcc tcagggaaac 180

cttccctgat tctccaaact gtaacagatt ccatatgcca atctctcata gctcagcact 240

ttatttgcag cacttactaa aagttatatt tatctataat ctggctatat gattatttga 300

tgactatctt tctctttcac tatactgtta gctctttaag agtaagcatt gtaagtgatg 360

gataccttaa ataccctact tgattgttac ccattctatg c 401

<210> 15
<211> 406
<212> DNA
<213> Homo sapiens

<220>
<221> variation
<222> 206
<223> /replace="t"

<400> 15
agccgtctac ataaaacagt tgaccttcaa gagggcattg gacagaaaga actggtggac 60

tttgatgact gatgggatgt agagaggagg aggccaggtt tcgaggtgga tggataccag 120

tggattactg acaggctgag tcagcaaggg gattggtggg agaaaaagct ggggttcagg 180

gtaaggcggc tgagaggtgg tataacatct agttagaggt atccagaaag caagtgaaaa 240

ctcaggattg gccttcagga gatgtattat ccattagcat gttcacgtat tcattcaatc 300

tctatacatt attaagcacc tgccgtgggg tgctactact gagtggccgc aggtgcgtgc 360

tgcagacatg gctaatctgt catggctgat agttatcaac cagtca 406

<210> 16
<211> 401
<212> DNA
<213> Homo sapiens

<220>
<221>  variation
<222>  201
<223>  /replace="c"

<400>  16
tgcagtcatg cccttaccat ctaatacaca gcagtttttc atcactgaca gaaaatgtac      60

aaccttaacc catcatgact tacagccctc catgtctgat cgtagcccca aacacaccat     120

acctttcaca tccgcactgc atccctgcat tgtaccacca gccagccacc ttcctcctcc     180

cttacccgct gggggatttg tacagtcgct gtagacccca gcctctgtgt gaccactact     240

ttgaagactt cctccttcac cctcttagga atttagcctt tctcacctaa tatttgtctc     300

cgctcattga acatgaagtc atggtttcta tgctggttgt tattctttct gttatttaca     360

gtgttttttt tgttgttgtt gttttttttt aagaaaagag t                         401

<210>  17
<211>  401
<212>  DNA
<213>  Homo sapiens

<220>
<221>  variation
<222>  201
<223>  /replace="g"

<400>  17
ccccacgggg ggggcctccg ggctgcacct gctcctggtt tgatttatga aaccagaata      60

cgatgtggag gcccacgatc gtcttccctg tacctgcagc cagagaatag gagattgtag     120

cccaccctgt gggtccctgt ggcccaaggc agcctgcggc tcccccggca tgggccctac     180

ctggtgggcc ctgaatgacc atgaaaggct tctccagagc ctccctgacc gccacgttct     240

ggctggggtt cagcttgtgg cggcctccgg ggatgttgta ggtctgccgc tccaggaacc     300

tgctggggat cactgagagg gggcagcctc cctgagcact catgcagggt ggggctgggg     360

gccagagggg aagctggagg ggtggggcag acctacctct g                         401

<210>  18
<211>  30
<212>  DNA
<213>  Artificial sequence

<220>
<223>  /note="Description of artificial sequence: primer"

<400>  18
acgttggatg tactccttgg ctctaatacc                                       30

<210>  19
<211>  30
<212>  DNA
<213>  Artificial sequence

<220>
<223>  /note="Description of artificial sequence: primer"

<400>  19
acgttggatg ggtgacaatg ctagccaatc                                        30

<210>  20
<211>  29
<212>  DNA
<213>  Artificial sequence

<220>
<223>  /note="Description of artificial sequence: primer"

<400>  20
acgttggatg actccttggc tctaatacc                                         29

<210>  21
<211>  30
<212>  DNA
<213>  Artificial sequence

<220>
<223>  /note="Description of artificial sequence: primer"

<400>  21
acgttggatg ggtgacaatg ctagccaatc                                        30

<210>  22
<211>  30
<212>  DNA
<213>  Artificial sequence

<220>
<223>  /note="Description of artificial sequence: primer"

<400>  22
acgttggatg acaatcctct tccctctctg                                        30

<210>  23
<211>  30
<212>  DNA
<213>  Artificial sequence

<220>
<223>  /note="Description of artificial sequence: primer"

<400>  23
acgttggatg atagacaagg gcactactcc                                        30

<210>  24
<211>  30
<212>  DNA
<213>  Artificial sequence

<220>
<223>  /note="Description of artificial sequence: primer"

<400>  24
acgttggatg gcttgagtgc taacatagac                                        30

<210>  25
<211>  30
<212>  DNA
<213>  Artificial sequence

<220>
<223>  /note="Description of artificial sequence: primer"

<400>  25
acgttggatg gccctatgaa gattagaccc                                        30

<210>  26
<211>  30
<212>  DNA
<213>  Artificial sequence

<220>
<223>  /note="Description of artificial sequence: primer"

<400>  26
acgttggatg gctctgtcta ctttgatctc                                        30

<210>  27
<211>  29
<212>  DNA
<213>  Artificial sequence

<220>
<223>  /note="Description of artificial sequence: primer"

<400>  27
acgttggatg gccttgtcat tacaaagcc                                         29

<210>  28
<211>  30
<212>  DNA
<213>  Artificial sequence

<220>
<223>  /note="Description of artificial sequence: primer"

<400>  28
acgttggatg ttggtgggag aaaaagctgg                                        30

<210>  29
<211>  30
<212>  DNA
<213>  Artificial sequence

<220>
<223>  /note="Description of artificial sequence: primer"

<400>  29
acgttggatg acttgctttc tggatacctc                                        30

<210>  30
<211>  30
<212>  DNA
<213>  Artificial sequence

<220>
<223>  /note="Description of artificial sequence: primer"

<400>  30
acgttggatg ttcgaaacca tgagatggtg                                        30

<210> 31
<211> 30
<212> DNA
<213> Artificial sequence

<220>
<223> /note="Description of artificial sequence: primer"

<400> 31
acgttggatg tttatttcct gggcccatcg 30


<210> 32
<211> 30
<212> DNA
<213> Artificial sequence

<220>
<223> /note="Description of artificial sequence: primer"

<400> 32
acgttggatg tttatttcct gggcccatcg 30


<210> 33
<211> 30
<212> DNA
<213> Artificial sequence

<220>
<223> /note="Description of artificial sequence: primer"

<400> 33
acgttggatg ttcgaaacca tgagatggtg 30


<210> 34
<211> 30
<212> DNA
<213> Artificial sequence

<220>
<223> /note="Description of artificial sequence: primer"

<400> 34
acgttggatg gaattgctcc tcagggaaac 30


<210> 35
<211> 30
<212> DNA
<213> Artificial sequence

<220>
<223> /note="Description of artificial sequence: primer"

<400> 35
acgttggatg gctgagctat gagagattgg 30


<210> 36
<211> 30
<212> DNA
<213> Artificial sequence

<220>
<223> /note="Description of artificial sequence: primer"

<400>  36
acgttggatg gaattgctcc tcagggaaac                                        30


<210>  37
<211>  30
<212>  DNA
<213>  Artificial sequence

<220>
<223>  /note="Description of artificial sequence: primer"

<400>  37
acgttggatg ctgagctatg agagattggc                                        30


<210>  38
<211>  30
<212>  DNA
<213>  Artificial sequence

<220>
<223>  /note="Description of artificial sequence: primer"

<400>  38
acgttggatg tctagaggat ctgcaacatc                                        30


<210>  39
<211>  30
<212>  DNA
<213>  Artificial sequence

<220>
<223>  /note="Description of artificial sequence: primer"

<400>  39
acgttggatg tgttaggggg tctggaggag                                        30


<210>  40
<211>  30
<212>  DNA
<213>  Artificial sequence

<220>
<223>  /note="Description of artificial sequence: primer"

<400>  40
acgttggatg tgttaggggg tctggaggag                                        30


<210>  41
<211>  30
<212>  DNA
<213>  Artificial sequence

<220>
<223>  /note="Description of artificial sequence: primer"

<400>  41
acgttggatg tctagaggat ctgcaacatc                                        30


<210>  42
<211>  30
<212>  DNA
<213>  Artificial sequence

<220>
<223> /note="Description of artificial sequence: primer"

<400> 42
acgttggatg tggagcagaa atttcatggg 30

<210> 43
<211> 30
<212> DNA
<213> Artificial sequence

<220>
<223> /note="Description of artificial sequence: primer"

<400> 43
acgttggatg tttgtccaca gtgaacctcc 30

<210> 44
<211> 30
<212> DNA
<213> Artificial sequence

<220>
<223> /note="Description of artificial sequence: primer"

<400> 44
acgttggatg tggagcagaa atttcatggg 30

<210> 45
<211> 30
<212> DNA
<213> Artificial sequence

<220>
<223> /note="Description of artificial sequence: primer"

<400> 45
acgttggatg tgtccacagt gaacctccag 30

<210> 46
<211> 31
<212> DNA
<213> Artificial sequence

<220>
<223> /note="Description of artificial sequence: primer"

<400> 46
acgttggatg gatctcatca aacctaactg c 31

<210> 47
<211> 29
<212> DNA
<213> Artificial sequence

<220>
<223> /note="Description of artificial sequence: primer"

<400> 47
acgttggatg aggaacaaac aacactaac 29

<210> 48
<211> 29
<212> DNA
<213> Artificial sequence

<220>
<223> /note="Description of artificial sequence: primer"

<400> 48
acgttggatg aggaacaaac aacactaac 29

<210> 49
<211> 31
<212> DNA
<213> Artificial sequence

<220>
<223> /note="Description of artificial sequence: primer"

<400> 49
acgttggatg gatctcatca aacctaactg c 31

<210> 50
<211> 30
<212> DNA
<213> Artificial sequence

<220>
<223> /note="Description of artificial sequence: primer"

<400> 50
acgttggatg aagctgaacc ccagccagaa 30

<210> 51
<211> 29
<212> DNA
<213> Artificial sequence

<220>
<223> /note="Description of artificial sequence: primer"

<400> 51
acgttggatg catgggccct acctggtgg 29

<210> 52
<211> 29
<212> DNA
<213> Artificial sequence

<220>
<223> /note="Description of artificial sequence: primer"

<400> 52
acgttggatg cctacctggt gggccctga 29

<210> 53
<211> 30
<212> DNA
<213> Artificial sequence

<220>
<223> /note="Description of artificial sequence: primer"

<400> 53
acgttggatg tgaaccccag ccagaacgtg 30

<210> 54
<211> 31
<212> DNA
<213> Artificial sequence

<220>
<223> /note="Description of artificial sequence: primer"

<400> 54
acgttggatg catctctgta ctatcttgac c 31

<210> 55
<211> 30
<212> DNA
<213> Artificial sequence

<220>
<223> /note="Description of artificial sequence: primer"

<400> 55
acgttggatg tagagaacag atacgaaacc 30

<210> 56
<211> 30
<212> DNA
<213> Artificial sequence

<220>
<223> /note="Description of artificial sequence: primer"

<400> 56
acgttggatg tagagaacag atacgaaacc 30

<210> 57
<211> 31
<212> DNA
<213> Artificial sequence

<220>
<223> /note="Description of artificial sequence: primer"

<400> 57
acgttggatg catctctgta ctatcttgac c 31

<210> 58
<211> 30
<212> DNA
<213> Artificial sequence

<220>
<223> /note="Description of artificial sequence: primer"

<400> 58
acgttggatg atttcttctg ccagacatcc 30

<210> 59
<211> 30
<212> DNA
<213> Artificial sequence

<220>
<223>  /note="Description of artificial sequence: primer"

<400>  59
acgttggatg tggtcatgtc ctaggacttc                                        30


<210>  60
<211>  30
<212>  DNA
<213>  Artificial sequence

<220>
<223>  /note="Description of artificial sequence: primer"

<400>  60
acgttggatg tctgccagac atccaaattc                                        30


<210>  61
<211>  30
<212>  DNA
<213>  Artificial sequence

<220>
<223>  /note="Description of artificial sequence: primer"

<400>  61
acgttggatg ggttgaattg tggtcatgtc                                        30


<210>  62
<211>  30
<212>  DNA
<213>  Artificial sequence

<220>
<223>  /note="Description of artificial sequence: primer"

<400>  62
acgttggatg ggatcattct ctggtgaggg                                        30


<210>  63
<211>  30
<212>  DNA
<213>  Artificial sequence

<220>
<223>  /note="Description of artificial sequence: primer"

<400>  63
acgttggatg caagtggcat caaatgcgtg                                        30


<210>  64
<211>  29
<212>  DNA
<213>  Artificial sequence

<220>
<223>  /note="Description of artificial sequence: primer"

<400>  64
acgttggatg actgcagggt gctgagcaa                                         29

<210>  65
<211>  30
<212>  DNA
<213>  Artificial sequence

<220>
<223>  /note="Description of artificial sequence: primer"

<400>  65
acgttggatg tcacaactgt tcggggtctg                                        30


<210>  66
<211>  30
<212>  DNA
<213>  Artificial sequence

<220>
<223>  /note="Description of artificial sequence: primer"

<400>  66
acgttggatg tgcattgtac caccagccag                                        30


<210>  67
<211>  30
<212>  DNA
<213>  Artificial sequence

<220>
<223>  /note="Description of artificial sequence: primer"

<400>  67
acgttggatg aagtagtggt cacacagagg                                        30


<210>  68
<211>  29
<212>  DNA
<213>  Artificial sequence

<220>
<223>  /note="Description of artificial sequence: primer"

<400>  68
acgttggatg caccttcctc ctcccttac                                         29


<210>  69
<211>  30
<212>  DNA
<213>  Artificial sequence

<220>
<223>  /note="Description of artificial sequence: primer"

<400>  69
acgttggatg tcaaagtagt ggtcacacag                                        30


<210>  70
<211>  31
<212>  DNA
<213>  Artificial sequence

<220>
<223>  /note="Description of artificial sequence: primer"

<400>  70
acgttggatg gggtgaaaaa gttgaaactc c                                    31


<210>  71
<211>  30
<212>  DNA
<213>  Artificial sequence

<220>
<223>  /note="Description of artificial sequence: primer"

<400>  71
acgttggatg gaggtgtgac acacttttcc                                      30


<210>  72
<211>  30
<212>  DNA
<213>  Artificial sequence

<220>
<223>  /note="Description of artificial sequence: primer"

<400>  72
acgttggatg gaggtgtgac acacttttcc                                      30


<210>  73
<211>  31
<212>  DNA
<213>  Artificial sequence

<220>
<223>  /note="Description of artificial sequence: primer"

<400>  73
acgttggatg gggtgaaaaa gttgaaactc c                                    31


<210>  74
<211>  29
<212>  DNA
<213>  Artificial sequence

<220>
<223>  /note="Description of artificial sequence: primer"

<400>  74
acgttggatg gtttcctctg cttcattaa                                       29


<210>  75
<211>  30
<212>  DNA
<213>  Artificial sequence

<220>
<223>  /note="Description of artificial sequence: primer"

<400>  75
acgttggatg agtcctgtat gctgatatcc                                      30


<210>  76
<211>  29
<212>  DNA
<213>  Artificial sequence

<220>
<223>  /note="Description of artificial sequence: primer"

<400>  76
acgttggatg tataagtttc ctctgcttc                                        29


<210>  77
<211>  30
<212>  DNA
<213>  Artificial sequence

<220>
<223>  /note="Description of artificial sequence: primer"

<400>  77
acgttggatg agtcctgtat gctgatatcc                                       30


<210>  78
<211>  30
<212>  DNA
<213>  Artificial sequence

<220>
<223>  /note="Description of artificial sequence: primer"

<400>  78
acgttggatg ttgccctcta aagcaattcc                                       30


<210>  79
<211>  30
<212>  DNA
<213>  Artificial sequence

<220>
<223>  /note="Description of artificial sequence: primer"

<400>  79
acgttggatg cacatgtacc ccttaactta                                       30


<210>  80
<211>  30
<212>  DNA
<213>  Artificial sequence

<220>
<223>  /note="Description of artificial sequence: primer"

<400>  80
acgttggatg agcagaagtg aggcaccttg                                       30


<210>  81
<211>  29
<212>  DNA
<213>  Artificial sequence

<220>
<223>  /note="Description of artificial sequence: primer"

<400>  81
acgttggatg agaaatcctt caaatgaaa                                        29

<210>  82
<211>  23
<212>  DNA
<213>  Artificial sequence

<220>
<223>  /note="Description of artificial sequence: primer"

<400>  82
gacgtaagga ccttaagatt agg                                              23


<210>  83
<211>  26
<212>  DNA
<213>  Artificial sequence

<220>
<223>  /note="Description of artificial sequence: primer"

<400>  83
ggtggcgtaa ggaccttaag attagg                                           26


<210>  84
<211>  22
<212>  DNA
<213>  Artificial sequence

<220>
<223>  /note="Description of artificial sequence: primer"

<400>  84
agtgctaaca tagacactca ta                                               22


<210>  85
<211>  20
<212>  DNA
<213>  Artificial sequence

<220>
<223>  /note="Description of artificial sequence: primer"

<400>  85
tgctaacata gacactcata                                                  20


<210>  86
<211>  25
<212>  DNA
<213>  Artificial sequence

<220>
<223>  /note="Description of artificial sequence: primer"

<400>  86
caagtcataa atgttagaag agggg                                            25


<210>  87
<211>  19
<212>  DNA
<213>  Artificial sequence

<220>
<223>  /note="Description of artificial sequence: primer"

<400>  87
cggctgagag gtggtataa                                                    19

<210>  88
<211>  24
<212>  DNA
<213>  Artificial sequence

<220>
<223>  /note="Description of artificial sequence: primer"

<400>  88
tggtgaaatt tggataaagt gacc                                              24

<210>  89
<211>  27
<212>  DNA
<213>  Artificial sequence

<220>
<223>  /note="Description of artificial sequence: primer"

<400>  89
ggcttccttc ctcaatccca tgtttta                                           27

<210>  90
<211>  17
<212>  DNA
<213>  Artificial sequence

<220>
<223>  /note="Description of artificial sequence: primer"

<400>  90
ccctgattct ccaaact                                                      17

<210>  91
<211>  25
<212>  DNA
<213>  Artificial sequence

<220>
<223>  /note="Description of artificial sequence: primer"

<400>  91
tccctcttcc ctgattctcc aaact                                             25

<210>  92
<211>  17
<212>  DNA
<213>  Artificial sequence

<220>
<223>  /note="Description of artificial sequence: primer"

<400>  92
tctgcaacat cagcaga                                                      17

<210>  93
<211>  17
<212>  DNA
<213>  Artificial sequence

<220>
<223>  /note="Description of artificial sequence: primer"

<400>  93
aggaacccac aggaagc                                                    17


<210>  94
<211>  24
<212>  DNA
<213>  Artificial sequence

<220>
<223>  /note="Description of artificial sequence: primer"

<400>  94
ggggaactag tgctgttaag aaaa                                            24


<210>  95
<211>  20
<212>  DNA
<213>  Artificial sequence

<220>
<223>  /note="Description of artificial sequence: primer"

<400>  95
aactagtgct gttaagaaaa                                                 20


<210>  96
<211>  25
<212>  DNA
<213>  Artificial sequence

<220>
<223>  /note="Description of artificial sequence: primer"

<400>  96
gcaaacctaa ctgcaagtgt tttcc                                           25


<210>  97
<211>  23
<212>  DNA
<213>  Artificial sequence

<220>
<223>  /note="Description of artificial sequence: primer"

<400>  97
caaacttcaa acatttgaaa aag                                             23


<210>  98
<211>  17
<212>  DNA
<213>  Artificial sequence

<220>
<223>  /note="Description of artificial sequence: primer"

<400>  98
tctggagaag cctttca                                                    17

<210>  99
<211>  23
<212>  DNA
<213>  Artificial sequence

<220>
<223>  /note="Description of artificial sequence: primer"

<400>  99
tttcgggtgg gccctgaatg acc                                              23


<210>  100
<211>  20
<212>  DNA
<213>  Artificial sequence

<220>
<223>  /note="Description of artificial sequence: primer"

<400>  100
gtactatctt gaccctggaa                                                  20


<210>  101
<211>  20
<212>  DNA
<213>  Artificial sequence

<220>
<223>  /note="Description of artificial sequence: primer"

<400>  101
tctttctctt ctaaagccca                                                  20


<210>  102
<211>  23
<212>  DNA
<213>  Artificial sequence

<220>
<223>  /note="Description of artificial sequence: primer"

<400>  102
ttaaattcat caccctctac ttc                                              23


<210>  103
<211>  22
<212>  DNA
<213>  Artificial sequence

<220>
<223>  /note="Description of artificial sequence: primer"

<400>  103
gaacttcatc accctctact tc                                               22


<210>  104
<211>  24
<212>  DNA
<213>  Artificial sequence

<220>
<223>  /note="Description of artificial sequence: primer"

<400>  104
aggcagggtg ctgagcaaca tccc                                              24


<210>  105
<211>  25
<212>  DNA
<213>  Artificial sequence

<220>
<223>  /note="Description of artificial sequence: primer"

<400>  105
ttttcagggt gctgagcaac atccc                                             25


<210>  106
<211>  21
<212>  DNA
<213>  Artificial sequence

<220>
<223>  /note="Description of artificial sequence: primer"

<400>  106
ccctacccgc tgggggattt g                                                 21


<210>  107
<211>  25
<212>  DNA
<213>  Artificial sequence

<220>
<223>  /note="Description of artificial sequence: primer"

<400>  107
cctcccttac ccgctggggg atttg                                             25


<210>  108
<211>  24
<212>  DNA
<213>  Artificial sequence

<220>
<223>  /note="Description of artificial sequence: primer"

<400>  108
caaactcctt gtcttttgta tgaa                                              24


<210>  109
<211>  25
<212>  DNA
<213>  Artificial sequence

<220>
<223>  /note="Description of artificial sequence: primer"

<400>  109
cccgtacaca cttttccaac tactg                                             25


<210>  110
<211>  26
<212>  DNA
<213>  Artificial sequence

<220>
<223>  /note="Description of artificial sequence: primer"

<400>  110
cttcattaat tttatgattg tgagag                                          26


<210>  111
<211>  26
<212>  DNA
<213>  Artificial sequence

<220>
<223>  /note="Description of artificial sequence: primer"

<400>  111
cgtcattaat tttatgattg tgagag                                          26


<210>  112
<211>  26
<212>  DNA
<213>  Artificial sequence

<220>
<223>  /note="Description of artificial sequence: primer"

<400>  112
ttccctctaa agcaattcct ttcttt                                          26


<210>  113
<211>  21
<212>  DNA
<213>  Artificial sequence

<220>
<223>  /note="Description of artificial sequence: primer"

<400>  113
tctaaagcaa ttcctttctt t                                               21

## Description

The present invention relates to a method of diagnosing a predisposition of a multiple sclerosis patient to become less susceptible or resistant to treatment with interferon β, said method comprising determining in a sample obtained from said patient the presence of one or more SNP(s) selected from the group consisting of SNPs defined by the sequences of SEQ ID NOs 1, 2, 3, 4, 5, 6, 7, 8, 9, 10, 11, 12, 13, 14, 15, 16 and 17, wherein the presence of one or more of said SNP(s) in said sample is indicative for said predisposition.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Multiple sclerosis (MS) is a chronic inflammatory disease of the central nervous system involving the destruction of myelin on neuronal axons (i.e. demyelination) which leads to the formation of multifocal plaques. MS can be divided into two categories: relapsing-remitting, and chronic-progressive. Chronic-progressive MS is further subdivided into primary-progressive, secondary-progressive, and progressive-relapsing.

Multiple sclerosis (MS) is the most common chronic inflammatory disease of the central nervous system in young adults. Over 1 million individuals worldwide are afflicted with MS. It's prevalence is estimated at 60 - 100 / 100.000 in the northern United States and northern Europe (see Kesselring Ed. "Epidemiologie." 3 ed. Psychiatrie, Neurologie, Klinische Psychologie. Stuttgart: Kohlhammer, 70-76, 1997). Although the mortality due to MS is low, neurological sequelae often occur early in life and prohibit private and professional development. Symptoms of MS include optic neuritis, fatigue, poor coordination, paresis, spasticity, dizziness, tremor, speech and swallowing difficulties, pain, emotional mood swings.

In about 80 to 90 % of patients, the disease starts with a clinically isolated syndrome which proceeds to a relapsing-remitting course. After several years there is an increasing tendency for the patient to enter a phase of slow, steady or fluctuating deterioration of neurologic function, which is then called a secondary progressive course. Only 10 - 15 % of patients demonstrate a primary progressive deterioration without any relapses just from the beginning.
The pathological hallmark of MS is the demyelinated plaque with relative axonal sparing and glial scar formation (Prineas "The Neuropathology of Multiple Sclerosis." Ed. J. C. Koetsier. Amsterdam: Elsevier Science Publishers, 213-257, 1985; Lassmann (1998) Mult Scler 4: 93-98). MS has been considered an autoimmune disorder caused by auto-reactive T cells and autoantibodies that drive an inflammatory process, leading to macrophage recruitment, and subsequent destruction of myelin/oligodendrocytes and axons. Recent detailed studies on large collections of MS lesions, however, have indicated that structural features of the plaques are extremely variable and the events involved in the immunopathogenesis of MS may be more complicated (Lucchinetti et al. (2000) Ann Neurol 47:707-717; Lassmann et al. (2001) Trends Mol Med 7: 115-121 A structural analysis of oligodendrocyte pathology demonstrated two principal patterns of oligodendrocyte pathology in MS lesions with oligodendrocyte survival or progenitor recruitment in the first and extensive destruction of myelinating cells in the second pattern. ln addition quite diverse patterns of myelin destruction were observed and grouped into four subtypes: (1) Macrophage-mediated demyelination; (2) Antibody-mediated demyelination; (3) distal oligodendrogliopathy and apoptosis; and (4) primary oligodendroglia degeneration (Lassmann et al. (2001) Trends Mol Med 7: 115-121). The finding of such heterogeneity is also supported by experimental data in animal models of MS.

The majority of MS cases is sporadic. However, about 15 % of the MS patients have an affected relative with the highest risk of recurrence being observed in siblings (Ebers et al. (1986) N Engl J Med 315: 1638-1642). In a large population-based study, it was found that almost 20 % of the index cases had an affected relative, again with the highest risk in siblings (Sadovnick et al. (1988) Am J Med Genet 29: 533-541). The case for heritability is supported also by studies in twins in whom one of each pair is known to have MS. Ebers et al. verified the diagnosis in 7 of 27 pairs of monozygotic twins (26 %) and in only one of 43 pairs of dizygotic twins (2.3 %). In two clinically normal monozygotic twins, lesions were detected by MRI, bringing the concordance rate to 33 % (Ebers et al. (1986) N Engl J Med 315: 1638-1642). The concordance rate in dizygotic pairs is similar to that in nontwin siblings. Within families with more than one affected member, no consistent genetic pattern has emerged. Studies dealing with the epidemiology and genetics of MS indicate multifactorial origin with environmental and genetic factors affecting disease susceptibility. (Sadovnick et al. (1988) Am J Med Genet 29: 533-541). Several genome screens have verified the association with HLA-DR2 and identified recently IL2RA and IL7RA as risk alleles for MS (The International Multiple Sclerosis Genetics Consortium (2007) N Engl J Med 357: 851-862; Gregory et al. (2007) Nat Genet 39: 1083-1091; Lundmark et al. (2007) Nat Genet 39: 1108-1113; Ramagopalan et al. (2007) N Engl J Med 357: 2199-2200; Weber et al. (2008) Genes lmmun. 9(3): 259-63).

There are presently four major categories of therapies for the treatment of MS: (1) interferon-β(IFNβ), which includes interferon-β-1a (IFNβ-1a), or interferon-β-1b (IFNβ-1b); (2) glatiramer acetate; (3) natalizumab; and (4) mitoxantrone. Several clinical trials have evaluated the various therapeutic options, for example, IFNβ, glatiramer acetate, immunoglobulins and mitoxantrone for patients suffering from different clinical subtypes of MS such as relapsing-remitting, primary or secondary-chronic progressive disease. Randomized, placebo-controlled, double-blind clinical trials with different preparations of IFNβ-1a (Avonex®, Rebif®) and IFNβ-1b (Betaferon®) were completed between 1993 and 1998 and resulted in the approval of these drugs for relapsing-remitting MS (RRMS). These studies found that IFNβ reduced relapse rates by approximately 30 % and inhibited new magnetic resonance imaging (MRI) lesions by approximately 70 % (Rudick et al. (2004) Ann Neurol 56: 548-55). Despite of extensive research, the exact mechanism of IFNβ in treatment of MS remains unclear. Several hypotheses have been proposed which are based on the suppression of lymphocyte proliferation, suppression of antigen presentation, suppression of migration of proinflammatory cells across the blood brain barrier, or a cytokine shift from TH1 to TH2 (Hartung et al. (2004) J Neurol 251: v12-v29).

IFNβ is the most common immunomodulatory therapy in MS. However, it is widely assumed that individual patients differ in their therapeutic response to IFNβ. Patients who continue to experience disease activity during therapy are termed non-responders, suboptimal responders, or breakthrough patients. Relapse rate and the Expanded Disability Status Scale (EDSS, Kurtzke (1983) Neurology 33:1444-1452) are often used as measurement of response. Criteria, however, vary among the different studies and lack validation. Response has been defined as having a lower relapse rate during IFNβ treatment compared with 1 to 2 years before therapy. A study of 262 patients from the European Database for Multiple Sclerosis found that 33 % of individuals were non-responders while the remaining individuals were classified as responders (Waubant et al. (2003) Neurology 61: 184-189). Another study found that half of the patients treated with INFβ demonstrates no benefit based on the EDSS as a measure of deterioration (Rio et al. (2002) Ann Neurol 52: 400-406). In another study new MRI lesion activity during IFNβ-1a treatment correlated with poor response to IFNβ-1a (Rudick et al. (2004) Ann Neurol 56: 548-555).
Many reports demonstrate the appearance of binding antibodies (BAB) specific for IFNβ during therapy with interferon drugs. A proportion of these BAB positive patients develop neutralising antibodies (NAB) specific for IFNβ. NABs were associated with a failure of lFNfl therapy in a lot of studies (Farrell et al. (2008) Mult Scler 14: 212-218; Boz et al. (2007) Mult Scler 13: 1127-1137; Tomassini et al. (2006) J Neurol 253: 287-293; Kappos et al. (2005) Neurology 65: 40-47; Francis et al. (2005) Neurology 65: 48-55). NABs to IFNβ reduce the therapeutic benefits measured by relapses and MRI activity (Francis et al. (2005) Neurology 65: 48-55). Data from the study of Kappos et al. also suggest that NABs to IFNβ-1a reduce treatment benefits as measured by change in Expanded Disability Status Scale score (Kappos et al. (2005) Neurology 65: 40-47). In contrast, BABs did not affect treatment benefits (Francis et al. (2005) Neurology 65: 48-55).
As a proportion of patients are only transiently positive for NABs, a second test is necessary after a three month interval. Patients who have remained NAB-negative during the first 24 months of therapy rarely developed NAB. On the contrary, the majority of patients, who had been NAB-positive from 12 through 30 months after start of therapy, remained NAB-positive (Sorensen et al. (2005) Neurology 65: 33-39). NAB against IFNβ, especially with high titers, tend to persist for a long time after discontinuation of IFNβ therapy (Petersen et al. (2006) Mult Scler 12: 247-252).
Relative affinity values were significantly higher in NAB-positive compared to NAB-negative samples and in samples of IFNβ-1a treated patients compared to IFNβ-1b. A significant positive correlation between relative affinity values and therapy duration indicates affinity maturation as another qualitative factor in IFNf3 neutralization (Gneiss et al. (2006) J. Neuroimmunol 174: 174-179). Therefore patients who become positive for BAB during IFNβ therapy are at risk to develop persisting NAB that are correlated with reduced efficacy and are a potential cause for renewed disease activity. Some smaller studies correlated the response to treatment with IFNβ to biological markers such as reduced IFNβ production (Petereit et al. (2002) Mult Scler 8: 492-494), decreased mitogen driven T cell proliferation (Killestein et al. (2002) J Neuroimmunol 133: 217-224), or the early and sustained induction of the tumour necrosis factor-related apoptosis-inducing ligand (TRAIL) (Wandinger et al. (2003) Lancet 361: 2036-2043). In a set of 70 selected genes, nine sets of gene triplets were identified which predicted response to therapy in 52 patients with 86 % accuracy (Baranzini et al. (2005) PLoS Biol 3: 166-176). Using microarray technology, non-responder and responder phenotypes to IFNβ were assessed by longitudinal gadolinium-enhanced MRI scans and clinical disease activity was shown to differ in their ex vivo gene expression profile (Stürzebecher et al. (2003) Brain 126: 1419-1429).

A long list of potential susceptibility gene candidates has been associated with MS. For example, polymorphisms in the interferon receptor 1 gene (IFNAR1) and interferon receptor 2 gene (IFNAR2) have been shown to confer susceptibility to MS, but not to predict response to IFNβ treatment (Leyva et al. (2005) J Neuroimmunol 163:165-171). It was recently demonstrated that the formation of antibodies against IFNβ is strongly associated with the HLA alleles DRB1*0401 and HLA-DRB*0408 (Hoffmann et al. (2008) The American Journal of Human Genetics 83: 219-227). Other potential gene candidates for MS susceptibility include the PRKCA, PTPRC, NOS2a, Ncf1, LAG3 and CD24 genes (Barton et al., (2004) Brain 127:1717-1722; Barcellos et al. (2004) Ann Neurol 55:793-800; Vyshkina et al (2004) Mult Scler 10:614-617; Hultqvist et al. (2004) Proc Natl Acad Sci USA 101:12646-12651; WO05/054810; WO03/031655). The growth factor TGF-β has also been proposed as a potential susceptibility gene for MS (Green et al (2001) J Neuroimmunol 116:116-124). However, several studies have shown that polymorphisms in TGF-β do not contribute in a major way to susceptibility to MS (He et al. (1998) J Neuroimmunol 86:13-19; McDonnell et al. (1999) Mult Scler 5:105-109; Weinshenker et al. (2001) J Neuroimmunol 120:138-145). Furthermore, polymorphisms in TGF-β have never been suggested for use in predicting response to IFNβ treatment.

Presently, the major histocompatibility complex (MHC) on the chromosome 6p region and the IL2RA and IL7RA genes are confirmed susceptibility loci for MS (The International Multiple Sclerosis Genetics Consortium (2007) N Engl J Med 357: 851-862; Gregory et al. (2007) Nat Genet 39: 1083-1091; Lundmark et al. (2007) Nat Genet 39: 1108-1113; Ramagopalan et al. (2007) N Engl J Med 357: 2199-2200; Weber et al. (2008) Genes lmmun. 9(3): 259-63). However, no differences between HLA-DR2 polymorphisms and the course of initial clinical symptoms or the distribution of responders and non-responders to IFNβ therapy was observed (Villoslada et al. (2002) J Neuroimmunol 130:194-201).

In summary, presently there exist no reliable methods to predict the appearance of BAB or NAB specific for IFNβ in an individual patient. An observation period of 1 to 2 years during therapy is necessary to classify the individual patient as antibody positive or negative. Furthermore, IFNβ therapy is associated with a number of adverse reactions, including flu-like symptoms, transient laboratory abnormalities, menstrual disorders, increased spasticity and injection site reactions. Therefore, there is a need for alternative or improved means and methods for assessing and predicting response to therapy of an MS patient.

A more efficient, individualized immunomodulatory therapy will not only be a major breakthrough for the large number of MS patients, but also lead to a dramatic cost reduction in the health system by decreasing hospitalization time or disease related disability.

Accordingly, the present invention relates in a first embodiment to a method of diagnosing a predisposition of a multiple sclerosis patient to become less susceptible or resistant to treatment with interferon β, said method comprising determining in a sample obtained from said patient the presence of one or more SNP(s) selected from the group consisting of SNPs defined by the sequences of SEQ ID NOs 1, 2, 3, 4, 5, 6, 7, 8, 9, 10, 11, 12, 13, 14, 15, 16 and 17, wherein the presence of one or more of said SNP(s) in said sample is indicative for said predisposition.

The term "MS" or "multiple sclerosis" has the meaning as established in the art and refers to a chronic inflammatory disease of the central nervous system involving the destruction of myelin on neuronal axons (i.e. demyelination) which leads to the formation of multifocal plaques. MS can be divided into two categories: relapsing-remitting, and chronic-progressive. Chronic-progressive MS is further subdivided into primary-progressive, secondary-progressive, and progressive-relapsing. Moreover, the further statements with regard to MS provided herein above in the background section are incorporated herein by reference.

The term "predisposition" as used in accordance with the invention relates to a genetic susceptibility of an MS patient to become less susceptible or resistant to treatment with interferon β. In connection with this embodiment of the present invention, the term relates preferably to the susceptibility to develop neutralizing antibodies and/or high titers of binding antibodies upon IFNβ treatment.

The predisposition is manifested as at least one alteration in the nucleic acid sequence of an individual having said predisposition as compared to that of an individual not having said predisposition. The occurrence of said alteration in the nucleic acid sequence is preferably correlated to the occurrence of anti-lFNβ antibodies, and a significant correlation indicates a predisposition to developing such antibodies.

The term "less susceptible to treatment with interferon β" as used herein means that a MS patient is less responsive for a treatment with interferon β as compared to a MS patient which is responsive for said treatment. The MS patient which is responsive for said treatment is preferably an MS patient which has been treated with IFNβ for less than 3 months, preferably less than 2 months, more preferably less than 1 month and most preferably for less than 1 week. Alternatively, the MS patient which is responsive for said treatment is preferably an MS patient which has no or no detectable IFNβ antibodies, which may be tested in a sample obtained from said patient, for example by an ELISA assay. In this regard, it is preferred with increasing preference that the patient is at least 25%, at least 50%, at least 75, at least 85%, at least 90%, or at least 95% less responsive to a treatment with interferon β as compared to a patient which is responsive for said treatment. In particular, the symptoms associated with multiple sclerosis of a MS patient are less susceptible to treatment by INFβ. Resistance or susceptibility to INFβ in a MS patient may be determined, e.g. by determining presence or absence, respectively, of relapses, or MRI activity as described in Francis et al (2005) Neurobiology 65: 48-55. Absence of relapses is indicative of susceptibility to treatment. A relapse is defined as the unprovoked and unanticipated occurrence of a new symptom associated with MS, or recurrence of an old symptom associated with MS, lasting for a period of greater than 24 hours. One or more relapses are indicative of resistance to IFNβ treatment. MRI activity is a magnetic resonance technique measuring multiple sclerosis signal changes in the brain that are due to changes of the neural activity caused by multiple sclerosis.

The term "resistant to treatment with interferon β" as used herein means that a patient is not responsive for a treatment with interferon β. In particular, in an MS patient the symptoms associated with multiple sclerosis are not treatable by INFβ.

The term "neutralizing antibodies" refers to antibodies the binding of which to their target antigen causes the neutralization of the biological effect of the molecule to which they bind. In the case of IFNβ, binding of neutralizing antibodies diminishes or completely abolishes the effect of IFNβ in the treatment of MS. MxA (Interferon-induced GTP-binding protein MxA) induced by IFN-β is used as a biological marker. (Pachner, A., 2003, Mol. Diagn. 7, 17-25). Another possibility is a validated cytopathic effect assay (P. S. Sorensen, Eur J Neurol. 2005 Nov;12(11):817-27, Hans-Peter Hartung J Neurol (2007) 54:827-837 2.)

The term "binding antibodies" refers to antibodies which specifically bind their target antigen. Binding antibodies may either be neutralizing antibodies as described herein above or antibodies solely binding to their target antigens without causing the neutralization of the biological effect of the molecule to which they bind. As regards the present invention the binding antibody preferably binds to IFNβ and thus may be specified as anti-IFNβ antibody or INFβ binding antibody.

ln this regard, high antibody titers may be determined by including defined positive and negative samples in an ELISA assay for determining antibody titers to obtain a standard. curve, for example as described in Francis et al (2005) Neurobiology 65: 48-55. The positive samples are obtained from MS patients which are less responsive or preferably resistant to treatment with IFNβ, whereas the negative samples were obtained from MS patient which are fully responsive to treatment with IFNβ. Whether a patient is resistant, less responsive or fully responsive to treatment with IFNβ may be determined by number, presence or absence, respectively of relapses, MxA induction or preferably by an ELISA assay. The obtained standard curve ranges from 100% (highest positive control) to 0% (no antibodies against IFNβ). Reactivity in an ELISA assay above 10%, preferably above 20%, more preferably above 25%, more preferably also above 30%, even more preferably above 40%, and most preferably above 50% of the highest positive control is then considered to be positive for high antibody titers as used herein

Accordingly, the term "high antibody titer against IFNβ" specifies with increasing preference an antibody titer above 10%, above 20%, above 25%, above 30%, above 40%, above 50%, above 60%, above 70%, above 80%, above 90%, or above 100% of the antibody titers of an MS patient which is resistant to IFNβ treatment, or alternatively of the average of more than one MS patient which is resistant to IFNβ treatment, preferably the average of more than 5, 10, 25, 50 or 100 MS patients which are resistant to IFNβ treatment.

IFNβ is a well known type I interferon. It is a cytokine and binds to a receptor designated as IFNα/β receptor or CD118 in humans. IFNβ is a glycoprotein which is - inter alia - secreted by fibroblasts. It is believed that immunomodulatory rather than antiviral or antiproliferative activity of interferon beta is responsible for disease amelioration. IFNβ suppress the MHC Class II expression, modify cytokine expression (e.g. reduce IFN-gamma and TNF-alpha production and elevate IL-10) and increase the production of chemokines and chemokine receptors. The mechanisms that is most important for the therapeutic effect remains unclear. (Sabine Cepok, ArchNeurol.2009;66(10):(doi:10.1001/archneurol.2009.138, Yong VW, Neurology. 1998 Sep;51 (3):682-9)

A "single nucleotide polymorphism (SNP)" is an alteration of a single nucleotide in a nucleic acid sequence making up the genetic information of an individual contained in a nucleic acid molecule. The term "nucleotide" is used herein interchangeably with the term "base". The SNP database maintained at NCBI (dbSNP; http://www.ncbi.nlm.nih.gov/SNP/ and Sayers et al. (2010) Nucleic Acids Research, Vol. 38, D5-D16, Database issue) divides SNPs into SNPs in the proximity of a known locus/gene and such that are 5' further away than 2 kb from the most 5' feature of a gene and 3' further away than 500 bases from the most 3' feature of a gene. SNPs in the proximity of a known locus are further divided into SNPs occurring at an mRNA location and such that do not. SNPs occurring in a mature mRNA are also referred to as coding SNP_{S}. The SNPs in accordance with the present invention comprise SNPs that are positioned in non-coding, e.g. intronic, regions within a known locus and SNPs that are found in intergenic regions. Each SNP is given a unique SNP identifier consisting of the letters "rs" followed by a specific number. With an SNP identifier the skilled person is in the position to retrieve any information relating to the SNP, such as, e.g., location of the SNP in the genome (chromosome, gene, specific sequence of the locus), functional annotation (if any) and the actual polymorphism, i.e. base exchange, at the given position within the wild type sequence. Besides the database maintained by the NCBI there exist further databases such as, e.g., F-SNP (http://compbio.cs.queensu.ca/F-SNP/), dbQSNP (http://qsnp.gen.kyushu-u.ac.jp/), CGAP SNP index (http://lpgws.nci.nih.gov/perl/snpbr) or GeneSNPs (http://www.genome.utah.edu/genesnps/). The Center for Human and Clinical Genetics of the Leiden University Medical Center hosts a website (http://www.humgen.nl/SNP_databases.html) that provides links to a variety of SNP-related websites such as, e.g., SNP databases, SNP-specific primer design software, SNP detection and effect prediction, SNP discovery tools or disease-causing variations. The data base entries in the NCBI SNP database which are defined by the rs-accession numbers provided herein show information as regards the position of the SNP relative to a reference sequence, wherein the reference sequence is the genomic DNA sequence at the respective human genetic locus..Also provided is information as regards the nature of the polymorphism, i.e. the base exchange, taking place at the SNP position expressed as wild type base/SNP base. For example, "C/T" means that at the SNP position the wild type sequence has a C while the presence of the SNP is indicated by a T at the same position. Furthermore and if applicable, information as regards the name of the gene the SNP is located in is provided. The above applies mutatis mutandis to other embodiments below.

The SNPs the presence of which is to be assessed in the method of the invention are associated with the predisposition of an MS patient to become less susceptible or resistant to a treatment with interferon β, preferably caused by the development of antibodies (preferably neutralizing antibodies and/or a high titer of anti-IFNβ antibodies) against IFNβ when receiving IFNβ in the course of MS therapy. In accordance with the present invention, the presence of one or more SNP(s), wherein the SNP(s) are selected from the group consisting of SNPs defined by the sequences of SEQ ID NOs 1, 2, 3, 4, 5, 6, 7, 8, 9, 10, 11, 12, 13, 14, 15, 16 and 17 is to be assayed for. Alternatively, the presence of the one or more SNP(s) being complementary to the nucleotide(s) of a sequence defined by the sequences of SEQ ID NOs 1, 2, 3, 4, 5, 6, 7, 8, 9, 10, 11, 12, 13, 14, 15, 16 and 17 is to be assayed for. It is of note that SEQ ID NOs 1, 2, 3, 4, 5, 6, 7, 8, 9, 10, 11, 12, 13, 14, 15, 16 and 17 show the sequences which are indicative for the predisposition as defined herein. In the case of SEQ ID NOs 1, 3, 9, 10, 11, 12, 13, 14, 15, 16 and 17 these sequences occur in a minority of the human population and are therefore also referred herein as minor allele (see Table 1). In the case of SEQ ID NOs 2, 4, 5, 6, 7 and 8 these sequences occur in a majority of the human population and are therefore also referred herein as major allele (see Table 1). The alternative base(s) at the polymorphic postion of SEQ ID NOs 1, 2, 3, 4, 5, 6, 7, 8, 9, 10, 11, 12, 13, 14, 15, 16 and 17 are described as the variation feature as set forth in the respective items 223 of the sequence listing. Presence of said alternative base is not indicative for the predisposition as defined herein. SEQ ID NOs 1, 3, 9, 10, 11, 12, 13, 14, 15, 16 and 17 having the alternative base are the major allele and SEQ ID NOs 2, 4, 5, 6, 7 and 8 having the alternative base are the minor allele (see Table 1).

The term "nucleotide" is well-known in the art and has the same meaning herein. Nucleotides are the building blocks of nucleic acid molecules.

SEQ ID NOs 1, 2, 3, 4, 5, 6, 7, 8, 9, 10, 11, 12, 13, 14, 15, 16 and 17 represent the following data base entries in the NCBI entrez SNP data base (http://www.ncbi.nlm.nih.gov/sites/entrez; update of January 8, 2009):

**Table 1:**

| CHR | SNP | SEQ ID | Position in SEQ ID | minor_allele | major_allele | minor_allele_frequency |
|---|---|---|---|---|---|---|
| 3 | rs184452 | 1 | 200 | G | T | 0.4229947 |
| 6 | rs2395175 | 3 | 201 | A | G | 0.1090067 |
| 6 | rs34369284 | 5 | 201 | T | C | 0.3480565 |
| 6 | rs9271300 | 7 | 201 | G | C | 0.4330357 |
| 6 | rs9272105 | 8 | 201 | A | G | 0.4419525 |
| 6 | rs2281389 | 2 | 201 | C | T | 0.1847643 |
| 6 | rs9496948 | 9 | 201 | C | T | 0.04688832 |
| 6 | rs300148 | 4 | 201 | C | T | 0.1597981 |
| 6 | rs4607409 | 6 | 200 | C | T | 0.1354430 |
| 8 | rs16912375 | 10 | 201 | A | G | 0.06139613 |
| 8 | rs4581054 | 11 | 201 | G | A | 0.06102694 |
| 8 | rs4961252 | 12 | 201 | G | A | 0.4067797 |
| 9 | rs1434302 | 13 | 201 | T | G | 0.06402116 |
| 11 | rs2344827 | 14 | 201 | G | A | 0.1556122 |
| 12 | rs1882545 | 15 | 206 | C | T | 0.3597884 |
| 15 | rs7183534 | 16 | 201 | T | C | 0.1253154 |
| 20 | rs3810481 | 17 | 201 | A | G | 0.1752577 |

As shown in the appended examples, all SNPs mentioned herein (SEQ ID NOs 1, 2, 3, 4, 5, 6, 7, 8, 9, 10, 11, 12, 13, 14, 15, 16 and 17) are strongly correlated with the formation of neutralizing antibodies against interferon in MS patients (P value less than 0.002). Out of these SNP_{S}, the SNPS represented by the sequence of SEQ ID NOs 3, 5, 6, 7, 8, 9, 12, 13, 16 and 17 are not only strongly correlated with the formation of neutralizing antibodies against interferon β in MS patients but are furthermore associated with high antibody titers of antibodies against interferon β in MS patients (P value less than 0.003). Accordingly, as regards the present invention, one or both parameters - high antibody titers against interferon and/or neutralizing antibodies against interferon-β - are indicative that a MS patient may become less susceptible or resistant to the treatment with interferon β.

In particular, the presence of high titers of antibodies against IFNβ was shown to be predictive of an increased risk for developing neutralizing antibodies. Accordingly and as demonstrated in the appended examples, the formation of high antibody titers is also indicative for the predisposition of non-responsiveness of MS patients to interferon β therapy. Similar to the determination of the presence of SNPs correlated with the formation of neutralizing antibodies, the determination of SNPs correlated with the formation of high antibody titers proves useful in determining the therapy of choice. The therapy of choice may involve one or more of (1) glatiramer acetate; (2) natalizumab; and (3) mitoxantrone, each of which may be given instead of INFβ in case a predisposition as described herein is diagnosed in a MS patient.

The term "sample" refers to a biological sample, such as, for example, cells, tissues, or fluids containing cells (including serum, whole blood, cerebrospinal fluid, lymph, saliva, milk, pus, urine) to be isolated from a MS patient that is or is not treated with IFNβ as a therapeutic measure or from cell culture constituents of a primary cell culture established from a tissue sample of said patient. A therapeutic regimen is the administration of IFNβ which is aimed at treating an MS patient's MS-related symptoms. Any tissue or liquid sample comprising cells obtained from said MS patient can be used for the determination of the presence of one or more SNP(s) or nucleotide(s) complementary thereto according to the invention. It is a well known fact that genomic DNA of individuals, which harbours the individual genetic makeup of all genes is obtainable from e. g. individual blood samples. Thus, a preferred sample in accordance with the invention is blood. Methods for preparing samples for genomic DNA extraction are well known in the art, and can be carried out using commercially available kits such as, for example, FlexiGene DNA Kit (Qiagen), or Qiagen Mini Prep Kit (Qiagen). In accordance with the method of diagnosing the predisposition of a multiple sclerosis patient to become less susceptible or resistant to treatment with interferon β of the present invention, the detection of any of the above-defined SNPs can be effected by several methods well-known in the art and described, e.g, in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989); Higgins and Hames (Eds.) "Nucleic acid hybridization, a practical approach" IRL Press Oxford, Washington DC, (1985); Nollau et al, Clin. Chem. 43 (1997), 1114-1128; Burczak and Mardis (Ed.), "Polymorphism Detection & Analysis Techniques", Eaton Pub Co, 2000; Cotton et al. (Ed.), "Mutation Detection: A Practical Approach", lrl Press, 1998; Taylor (Ed.), "Laboratory Methods for the Detection of Mutations and Polymorphisms in DNA", CRC Press, 1997; Taylor and Day (Ed.) "Guide to Mutation Detection", Wiley, 2005. For example, hybridization based methods (ltakura et al., Ann Rev Biochem (1984), 53; 323-356) or PCR based methods can be employed. Preferred methods will be described in detail further below.

The present inventors have surprisingly found that single nucleotide polymorphisms located in non-coding regions of the genetic information are associated with the appearance of antibodies specific for IFNβ in MS patients treated with IFNβ. More particularly, the SNPs found in the course of the present invention are associated with the development of neutralizing antibodies, i.e. those antibodies which neutralize the desired effect of IFNβ on the immune system of the MS patient. A subset of said SNPs is associated with high titers of INFβ-binding antibodies in a MS patient. The large group of MS patients observed in the course of the present invention allows the determination of said SNPs with a high statistical significance.

The SNP positions identified are to the best knowledge of the inventors not related to genes encoding proteins involved in the regulation of the expression or activity of IFNβ. Thus, it is surprising that these apparently unrelated SNPs can determine the responsiveness of an MS patient to an IFNβ therapy. Until the present invention, MS patients were exposed to a (long) IFNβ therapy without knowing whether he/she will develop antibodies against IFNβ. The present invention now enables for the first time to determine whether it is sensible to apply an IFNβ therapy instead of one of the other three therapeutic options presently available (see above).

The assessment of the presence of one or more of the above SNP(s) or the respective complementary nucleotide(s) allows for the prediction of the responsiveness. The more of the above-mentioned SNPs or corresponding complementary nucleotides thereof are present, the more significant is the result, i.e. the higher is the chance that the predicted responsiveness occurs upon treatment with IFNβ. Thus, it is preferred that the presence of more than one SNP or respective complementary nucleotide thereof be assessed for. Accordingly, the assessment of the presence of at least 2, more preferably at least 3, at least 4, at least 5, at least 6, at least. 10, or at least 15 of the above SNPs or their corresponding complementary nucleotides are more preferred embodiments in accordance with the invention. Further, any possible combination of SNPs or corresponding complementary nucleotides thereof to be assessed for is explicitly envisaged, even if not explicitly mentioned.

The identification of polymorphisms associated with the development of antibodies against IFNβ is important for the prediction of the onset, the clinical course and/or severity of symptoms which are intimately associated with the therapy outcome. Therefore, assessment of the presence of one or more of the SNP(s) or corresponding complementary nucleotide(s) thereof as described herein and associated with the development of antibodies against IFNβ in an MS patient, is a valuable tool for designing an individual therapeutic strategy to treat MS patients which due to the present invention has now become possible.

In another embodiment the present invention relates to a method of diagnosing a predisposition of a multiple sclerosis patient for developing neutralizing antibodies against interferon-β, said method comprising determining in a sample obtained from said patient the presence of one or more SNP(s) selected from the group consisting of SNPs defined by the sequences of SEQ ID NOs 1, 2, 3, 4, 5, 6, 7, 8, 9, 10, 11, 12, 13, 14, 15, 16 and 17, wherein the presence of one or more of said SNP(s) in said sample is indicative for said predisposition.

ln a preferred embodiment the described method of the invention further comprises the step of diagnosing a predisposition of said patient for developing high antibody titers against interferon-β, wherein the one or more SNP(s) are selected from a subgroup consisting of SNPs defined by the sequences of SEQ ID NOs. 3, 5, 6, 7, 8, 9, 12, 13, 16 and 17, wherein the presence of one or more of said SNP(s) selected from said subgroup in said sample is indicative for both said predispositions.

In a further embodiment, the invention relates to a method of diagnosing a predisposition of a multiple sclerosis patient for developing high antibody titers against interferon-β, said method comprising assessing in a sample obtained from said patient the presence of one or more SNP(s) selected from the group consisting of SNPs defined by the sequences of SEQ ID NOs 3, 5, 6, 7, 8, 9, 12, 13, 16 and 17; wherein the presence of one or more of said SNP(s) in said sample is indicative for said predisposition.

In a preferred embodiment of the methods of the invention described herein above said one or more SNP(s) are selected from the group consisting of SNPs defined by the sequences of SEQ ID NOs 5, 8 and 12.

SEQ ID NOs 5, 8 and 12 represent the sequences of only those SNPs that are statistically highly significantly associated with the predisposition as described herein (p .< 5e-08). The larger list of SNPs represented by SEQ ID NOs 1, 2, 3, 4, 5, 6, 7, 8, 9, 10, 11, 12, 13, 14, 15, 16 and 17 is the list of all SNPs that pass a threshold of 0.003. As it is evident from the examples provided herein, significance analysis was done using a generalized linear model, including adjustment for age, sex, the type of interferon and the subtype of multiple sclerosis.

As shown in the appended examples, SNPs of SEQ ID NOs 5, 8 and 12 are most significantly associated with the formation of high antibody titers against interferon in MS patients.

In a further preferred embodiment of the methods of the invention described herein, the presence of at least two SNPs selected from (each of) said group(s) is determined.

As will be readily appreciated by the skilled person, the assessment for the presence of at least two of SNPs from (each of) said group(s) described above is even more predictive for the formation of a predisposition as described above. In more detail, at least two of SNPs represented by SEQ ID NOs 1, 2, 4, 10, 11, 14 and 15 and in addition at least two SNPs represented by SEQ ID NOs 3, 5, 6, 7, 8, 9, 12, 13, 16 and 17 may be assessed. Furthermore, at least two of SNPs represented by SEQ ID NOs 1, 2, 3, 4, 5, 6, 7, 8, 9, 10, 11, 12, 13, 14, 15, 16 and 17; at least two of SNPs represented by SEQ ID NOs 3, 5, 6, 7, 8, 9, 12, 13, 16 and 17; or at least two of SNPs represented by SEQ ID NOs 5, 8 and 12 may be assessed.

Accordingly, the assessment for the presence of any combination of at least one SNP indicative of the formation of neutralizing antibodies with at least one SNP indicative of the formation of high antibody titers is explicitly envisaged in the present invention. In particular, in addition to the assessment for the presence of at least one, preferably at least two, at least three, at least four, at least five or more SNPs indicative for the formation of neutralizing antibodies, the presence of at least one, preferably at least two, at least three, at least four, at least five or more SNPs indicative for the formation of high antibody titers can be assessed for to arrive at an even more reliable prediction of the patient's responsiveness to interferon.

In a preferred embodiment, the determining of the presence of one or more of the SNP(s) of (a) or the respective complementary nucleotide(s) of (b) is effected by any one of
(a) an assay based on physical separation of nucleic acid strands,
(b) ligase chain reaction assay,
(c) cleavage and digestion assay,
(d) sequencing assay,
(e) nucleic amplification assay, or
(f) hybridization assay.

Examples for assays based on physical separation of nucleic acid molecules include without limitation MALDI-TOF, HPLC, denaturing gradient gel electrophoresis (DGGE) and other such methods known in the art, see for example Petersen et al., Hum. Mutat. 20 (2002) 253-259; Hsia et al., Theor. Appl. Genet. 111 (2005) 218-225; Tost and Gut, Clin. Biochem. 35 (2005) 335-350; Palais et al., Anal. Biochem. 346 (2005) 167-175.

The principle of ligase chain reaction (LCR) assays is based in part on the ligation of two adjacent synthetic oligonucleotide primers, which uniquely hybridize to one strand of the target DNA. The junction of the two primers is usually positioned so that the nucleotide at the 3' end of the upstream primer coincides with a potential single base-pair difference in the targeted sequence potentially defining polymorphisms correlated to a given phenotype. If the target nucleotide at that site complements the nucleotide at the 3' end of the upstream primer, the two adjoining primers can be covalently joined by the ligase. The unique feature of LCR is a second pair of primers, almost entirely complementary to the first pair, that are designed with the nucleotide at the 3' end of the upstream primer denoting the sequence difference. In a cycling reaction, using a thermostable DNA ligase, both ligated products can then serve as templates for the next reaction cycle, leading to an exponential amplification process analogous to PCR amplification. If there is a mismatch at the primer junction, it will be discriminated against by thermostable ligase and the primers will not be ligated. The absence of the ligated product therefore indicates at least a single base-pair change in the target sequence. The method is reviewed e. g. in Wiedmann et al. (1994), Landegren (1993) and Barany (1991). Other equally suitable methods based on the utilization of ligases in the detection of SNPs are e. g. ligase detection reaction (LDR), pLCR or gapped LCR.

Examples for cleavage and digestion assay include without limitation restriction digestion assays such as restriction fragments length polymorphism assays (RFLP assays) of DNA contained in a sample obtained from a patient, assays based on chemical cleavage methods and enzyme mismatch cleavage assays, see e.g. Youil et al., Proc. Natl. Acad. Sci. U.S.A. 92 (1995) 87-91; Todd et al., J. Oral Maxil. Surg. 59 (2001) 660-667; Amar et al., J. Clin. Microbiol. 40 (2002) 446-452. Optionally the DNA may be amplified prior to analysis, the methods of which are well known in the art.

Examples for nucleic acid amplification assay and means to perform such include without limitation PCR, (including nested PCR, RT-PCR, PCR extension assays, Nucleic Acid Sequence Base Amplification (NASBA), single-strand confirmation polymorphism (SSCP) PCR, solid phase PCR), amplification refractory mutation systems (ARMSTM) and amplification refractory mutation system linear extension (ALEXTM) assays. Details of such methods can be found in art, see, for example, Newton et al., Nucleic Acids Res. 17 (1989) 2503-2516; Agrawal (Ed.), "Protocols for Oligonucleotides and Analogs: Synthesis and Properties (Methods in Molecular Biology, 20)", Humana Press, 1993; Haque et al., Diagn. Mol. Pathol. 7 (1998) 248-252; Innis et al. (Ed.), "PCR Applications: Protocols for Functional Genomics", Academic Press, 1999; Chen and Janes (Ed.), "PCR Cloning Protocols: From Molecular Cloning to Genetic", 2nd edition, Humana Press, 2002; Pissardet al., Clin. Chem. 48 (2002) 769-772; Steemers et al., Nature Meth. 3 (2006) 31-33; Kakavas et al., J. Clin. Lab. Anal. 20 (2006) 1-7.

PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e.g. 1 min at 72°C, depending on the length of DNA template and the enzyme used), denaturing (e.g. 10 s at 94°C) and a final annealing step at 55°C for 1 min as well as a final extension step at 72°C for 5 min. Suitable polymerases for use with a DNA template include, for example, E. coli DNA polymerase l or its Klenow fragment, T4 DNA polymerase, Tth polymerase, Taq polymerase, a heat-stable DNA polymerase isolated from Thermus aquaticus, Vent, Amplitaq, Pfu and KOD, some of which may exhibit proof-reading function and/or different temperature optima. However, the person skilled in the art knows how to optimize PCR conditions for the amplification of specific nucleic acid molecules with primers of different length and/or composition or to scale down or increase the volume of the reaction mix.

A PCR based method to detect SNPs, for example, uses primers, wherein one primer hybridizes specifically to the region on the target sequence carrying the SNP and wherein said primer is exactly complementary to the target sequence. According to that method applied to the present invention, amplification of the target sequence will only occur, if said target sequence carries an SNP or a corresponding complementary nucleotide thereof as defined herein above. This is because the nucleic acid molecule, i.e. the primer, will under stringent hybridization conditions not hybridize to the wild type sequence (with the consequence that no amplification product is obtained) but only to the sequence carrying said SNP. The term "wild type sequence" as used herein refers to the nucleotide sequence at the locus of the respective SNP or corresponding complementary nucleotide thereof not displaying the nucleotide polymorphism characteristic of said SNP.

Said locus may display the nucleotide of the respective wild type reference sequence as provided herein and described above for each SNP or corresponding complementary nucleotide thereof (cf. nucleotide at position (WT/SNP)). In one embodiment, the complementary portion of the oligo- or polynucleotide containing the SNP is arranged in said primer in a central position; "central" meaning most preferably that an equal number of nucleotides are located in the 5'- and 3'- direction adjacent to the position of the SNP. In different embodiments (each about) 100, 90, 80, 70 and 60 percent of nucleotides are located in the 5'- or 3'- direction adjacent to the position of the SNP and remaining nucleotides are located in the opposite direction. Also encompassed are embodiments where the primer sequence ends in the position complementary to the SNP. Of course, primers complementary to the wild type sequence can also be used wherein an amplification product is indicative of a situation where no anti-lFNβ-associated nucleotide exchange is present.

Also envisaged is the use of a primer extension assay wherein the target sequence is annealed to a primer complementary to the region adjacent to the SNP site. Dideoxyribonucleotides (ddNTPs) and DNA polymerase are added to the mixture and the primer is extended by a single nucleotide. The single nucleotide added is dependent on the allele of the amplified DNA. Primer extension biochemistry can be coupled with a variety of detection schemes, comprising fluorescence, fluorescence polarization (FP), luminescence and mass spectrometry (MS) (Li et al., Electrophoresis (1999), 20; 6, 1258-1265). Primers can be designed by methods well-known in the art including, for example, publicly available programs as primer3 (University of Massachusetts Medical School, U.S.A. (http://frodo.wi.mit.edu/)), Primer-Blast (National Library of Medicine (NLM) web pages (http://www.ncbi.nlm.nih.gov/tools/primer-blast/)), NetPrimer (PREMIER Biosoft International web pages (http://www.premierbiosoft.com/netprimer/)), Primer fox (http://www.primerfox.com/ by Stephan Fuchs (Ernst-Moritz-Arndt-University Greifswald)), IDT PrimerQuest (Integrated DNA Technologies (IDT) web pages (http://eu.idtdna.com/Scitools/Applications/Primerquest/), Primo Pro (Chang Bioscience, Inc. web pages (http://www.changbioscience.com/primo/primo.html)), Primers can also designed manually, following guidelines as mentioned for example on the web pages mentioned above or in the following references: Sharrocks, A.D., The design of primers for PCR, in PCR Technology, Current Innovations, Griffin, H.G., and Griffin, A.M, Ed., CRC Press, London, 1994, 5-11; Dieffenbach, C.W., Lowe, T.M.J., Dveksler, G.S., General Concepts for PCR Primer Design, in PCR Primer, A Laboratory Manual, Dieffenbach, C.W, and Dveksler, G.S., Ed., Cold Spring Harbor Laboratory Press, New York, 1995, 133-155.

Preferred primers to be used according to the methods of the invention are primers that specifically hybridize to the nucleotide sequences having SEQ ID NO 1 to 17 or their reverse complement. Also envisaged are primers that amplify nucleotide sequences having SEQ ID NO 1 to 17 or their reverse complement without specifically hybridizing thereto.

Examples for sequencing assays comprise without limitation approaches of sequence analysis by direct sequencing, fluorescent SSCP in an automated DNA sequencer and pyrosequencing. These procedures are common in the art, see e.g. Adams et al. (Ed.), "Automated DNA Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422. Furthermore, the genomic DNA obtained from the subject may be sequenced to identify SNPs or corresponding complementary nucleotides as described herein above.

Examples for hybridization assays comprise without limitation Northern and Southern blot assays, heteroduplex analysis, detection of mutations by sequence specific oligonucleotide hybridization, allele-specific oligonucleotide hybridization on DNA chips, assays based on the Illumina's^{®} technology, assays based on the BeadArray^{®} technology, see, for example, Barnes et al., Nucleic Acids Res. 33 (2005) 5914-5923; Fan et al., Biotechniques 39 (2005) 583-588; Shen et al., Mutat. Res.-Fund. Mol. M. 573 (2005) 70-82; Steemers and Gunderson, Pharmacogenomics, 6 (2005) 777-782.

The nucleic acid molecules used as probes for hybridization can, of course, be conveniently labeled by incorporating or attaching, e.g., a radioactive or other marker. Such markers are well known in the art. The labeling of said nucleic acid molecules can be effected by conventional methods. Specific hybridization of the above mentioned probes or primers preferably occurs at stringent hybridization conditions. The establishment of suitable hybridization conditions is referred to in standard text books such as Sambrook, Russell "Molecular Cloning, A Laboratory Manual", Cold Spring Harbor Laboratory, N.Y. (2001 ); Ausubel, "Current Protocols in Molecular Biology", Green Publishing Associates and Wiley Interscience, N.Y. (2002), or Higgins and Hames (Eds.) "Nucleic acid hybridization, a practical approach" IRL Press Oxford, Washington DC, (1985).

The term "stringent hybridization conditions" refers to hybridization conditions under which nucleic acid molecules such as, e.g., the nucleic acid molecules described herein, are capable of hybridizing to target DNA sequences or parts thereof and do not cross-hybridize to unrelated, non-target DNA sequences. In particular with regard to hybridization probes it is preferred that the hybridization conditions are stringent to allow for the detection of single base mismatches. Appropriate stringent hybridization conditions for each nucleic acid sequence may be established by a person skilled in the art on well-known parameters such as temperature, composition of the nucleic acid molecules (GC-content), salt conditions etc.; see, for example, Sambrook et al., "Molecular Cloning, A Laboratory Manual"; CSH Press, Cold Spring Harbor, 1989 or Higgins and Hames (eds.), loc. cit., see in particular the chapter "Hybridization Strategy" by Britten & Davidson, 3 to 15. Such conditions comprise e.g. an overnight incubation at 65°C in 4x SSC (600 mM NaCl, 60 mM sodium citrate) followed by washing at 65°C in 0.1x SSC for one hour. Alternatively, hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. In general, for achieving "high stringency", salt concentrations of 0.0165 to 0.033 M and a temperature of 5 to 10°C below the melting temperature (Tm) of the nucleic acid are sufficient. Only sequences with a high degree of identity will hybridize under these conditions. Preferred is that hybridization conditions are chosen that allow hybridization only of nucleic acid molecules with complete identity to the target DNA sequence in order to detect single base mismatches.

It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Changes in the stringency of hybridization are primarily accomplished through the manipulation of formamide concentration (lower percentages of formamide result in lowered stringency), salt conditions (lower salt concentrations result in lowered stringency), or temperature. Such modifications can generally be effected by the skilled person without further ado.

A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed) as described infra.

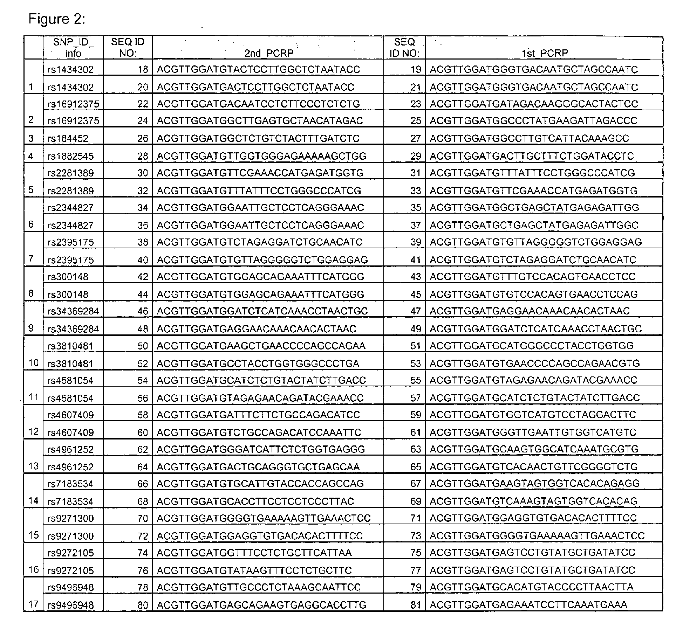

In a more preferred embodiment of the method of the invention, the nucleic acid amplification assay is a PCR assay making use of one or more forward primer(s) and one or more reverse primer(s), wherein at least one primer is identical or complementary to a region of a sequence of any one of SEQ ID NOs 1, 2, 3, 4, 5, 6, 7, 8, 9, 10, 11, 12, 13, 14, 15, 16 and 17 comprising position 200 of SEQ ID NOs 1 and 6, position 201 of SEQ ID NOs 2, 3, 4, 5, 7, 8, 9, 10, 11, 12, 13, 14, 15, 16 and 17, and position 206 of SEQ ID NO 15.
In this regard, it is preferred that the one or more forward primer(s) and the one or more reverse primer(s) of the PCR assay comprise one or more primer pairs selected from the group of primer pairs represented by the pairs of nucleic acid molecule sequences having SEQ ID NOs: 18/19, 20/21, 22/23, 24/25, 26/27, 28/29, 30/31, 32/33, 34/35, 36/37, 38/39, 40/41, 42/43, 44/45, 46/47, 48/49, 50/51, 52/53, 54/55, 56/57, 58/59, 60/61, 62/63, 64/65, 66/67, 68/69, 70/71, 72/73, 74/75, 76/77, 78/79 and 80/81 (see Figure 2).

The term "nucleic acid molecule", in accordance with the present invention, comprises polynucleotides and oligonucleotides, wherein the term "polynucleotide" defines a nucleic acid molecule consisting of more than 50 nucleotides. The group of molecules subsumed under "polynucleotide" also comprises complete genes. Also included by said definition are vectors such as cloning and expression vectors. The term "oligonucleotide" describes in the context of the invention nucleic acid molecules consisting of less than 50, preferably less than 45, more preferably less than 40, even more preferably less than 35 and most preferably less than 31 nucleotides. The term "oligonucleotide" describes in the context of the invention nucleic acid molecules consisting with increasing preference of more than 12, 13, 14, 15, 16, 17, 18, 20, 22 or 25 nucleotides. The primer of the invention is preferably an oligonucleotide. The probe of the invention is preferably a "polynucleotide". The probe of the is preferably between 100 and 5000 nucleotides, more preferably between, 200 and 2000 nucleotides and most preferably between 300 and 500bp. The nucleic acid molecules to be used in the invention include DNA, such as cDNA or genomic DNA, RNA (e.g. mRNA), also in synthetic or semisynthetic form, further synthetic or semisynthetic derivatives of DNA or RNA (e.g. PNA or phosphorothioates) and mixed polymers, both sense and antisense strands. They may contain additional non-natural or derivatized nucleotide bases, as will be readily appreciated by those skilled in the art. Synthetic or semisynthetic derivatives of DNA or RNA are, of course, not comprised in a sample derived from a patient, but may be used as detective means in the method of the invention.

For the purposes of the present invention, a peptide nucleic acid (PNA) is a polyamide type of DNA analog and the monomeric units for the derivatives of adenine, guanine, thymine and cytosine are available commercially (Perceptive Biosystems). Certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (T.sub.m) by 8°-20°C, vs. 4°-16°C for the DNA/DNA 15-mer duplex. Also, the absence of charge groups in PNA means that hybridization can be done at low ionic strengths and reduce possible interference by salt during the analysis.

In another more preferred embodiment of the method of the invention, the the nucleic acid amplification assay is a primer extension assay making use of one or more primer(s), wherein at least one primer is identical or complementary to a region of a sequence of any one of SEQ ID NOs 1, 2, 3, 4, 5, 6, 7, 8, 9, 10, 11, 12, 13, 14, 15, 16 and 17 immediately upstream of position 200 of SEQ ID NOs 1 and 6, position 201 of SEQ ID NOs 2, 3, 4, 5, 7, 8, 9, 10, 11, 12, 13, 14, 15, 16 and 17, and position 206 of SEQ ID NO 15.

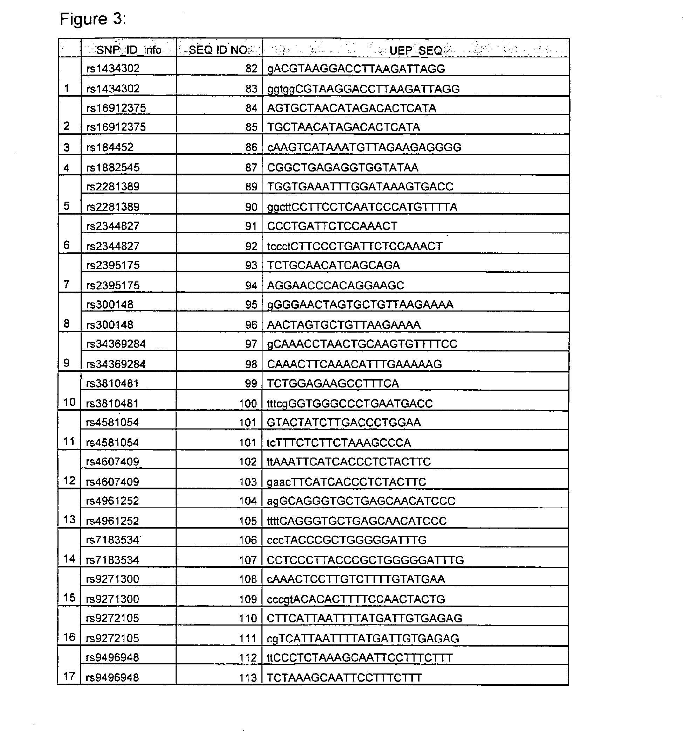

In this regard, it is preferred that the of one or more primer(s) of the primer extension assay comprise one or more primers selected from the group of primers represented by the nucleic acid molecule sequences having SEQ ID NOs 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112 and 113 (see Figure 3).

In a more preferred embodiment, the hybridization assay makes use of one or more nucleic acid molecule(s) as probe(s), wherein at least one probe is identical or complementary to a region of a sequence of any one of SEQ ID NOs: 1, 2, 3, 4, 5, 6, 7, 8, 9, 10, 11, 12, 13, 14, 15, 16 and 17 comprising position 200 of SEQ ID NOs 1 and 6, position 201 of SEQ ID NOs 2; 3, 4, 5, 7, 8, 9, 10, 11, 12, 13, 14, 15, 16 and 17, and position 206 of SEQ ID NO 15..

In .this regard, it is preferred that the one or more nucleic acid molecule(s) used as probes in the hybridization assay comprise with increasing preference at least 5 to 1000 nucleotides, 10 to 500 nucleotides, 15 to 350, or 20-200 nucleotides.

In another embodiment, the invention provides a solid support comprising one or more of the primers or probes recited herein above.

A solid support according to the invention provides a surface for the attachment of the one or more of the nucleic acid molecules referred to herein above. Said surface according to the invention may be any surface suitable for attaching said nucleic acid molecules. The surface may be a coating applied to the support or carrier, or the surface of the support or carrier itself may be used. Support or carrier materials commonly used in the art are envisaged for the purpose of the present invention such as, e.g., glass, plastic, gold or silicon. Coatings according to the invention, if present, include, e.g., poly-L-lysine- and amino-silane-coatings as well as epoxy- and aldehyde-activated surfaces. The nucleic acid molecules may, e.g., be attached to beads or to the walls of a reaction container, such as in solid phase PCR. The attachment of the nucleotide molecules of the invention may involve chemical modification of the molecules that are well-known in the art such as, e.g., phosphorylation.

In a preferred embodiment said solid support is a DNA chip. Methods for the production of DNA chips as well as the use of DNA chip technology are well known in the art and described e.g. in Lohara et al., Nucleic Acids Res. 30 (2002) e87; Flavell et al., Nucleic Acids Res. 31 (2003) e115; Gunderson et al., Nature Genetics 37 (2005) 549-554. It is readily appreciated by the skilled person that attaching the nucleic acid molecules to a solid support provides the platform for a variety of diagnostic approaches with regard to devising diagnostic apparatuses or ready-to-use diagnostic kits.

Further, the invention relates to a diagnostic composition for diagnosing a predisposition of a multiple sclerosis patient to become less susceptible or resistant to treatment with interferon β, said composition comprising one or more of the primer(s), one or more of the probe(s), and/or one or more of the solid support(s) of of the invention.

The term "composition", as used in accordance with the present invention, relates to a composition which comprises at least one or more of the nucleic acid molecules described herein above. It may, optionally, comprise further molecules capable of altering the characteristics of the nucleic acid molecules described herein thereby, for example, suppressing, stabilizing, blocking, modulating and/or activating their function. The composition may be in solid, liquid or gaseous form and may be, inter alia, in the form of (a) powder(s), (a) tablet(s), (a) solution(s) or (an) aerosol(s). Diagnostic compositions are well-known to the person skilled in the art as well as their fabrication and use for diagnosing the target condition. The diagnostic composition of the present invention provides for reliable and repeatable diagnosis of a predisposition of a MS patient for responsiveness to a treatment of MS by administration of lFNβ.

In a preferred embodiment, the genetic background of the MS patient is Caucasian.

In a different embodiment, the present invention relates to a kit comprising the solid support of the invention or the diagnostic composition of the invention in one or more containers and, optionally, instructions for using said kit or diagnostic composition.

The various components of the kit may be packaged in one or more containers such as one or more vials. The vials may, in addition to the components, comprise preservatives or buffers for storage. The instructions for using the kit or diagnostic composition of the invention preferably comprise instructions how to perform the methods of the invention.

The figures show:
Figure 1: The ROC curve of the classifier listed in Example 1.
Figure 2: Primer pairs preferably used in the PCR assay described herein.
Figure 3: Primers preferably used in the primer extension assay described herein.

The examples illustrate the invention

### Example 1: Polymorphisms can be used to predict the appearance of IFNβ neutralising antibodies in MS patients treated with interferon drugs

390 patients diagnosed with MS were selected according to their antibody response to interferon treatment. 142 patients responded with neutralizing antibodies to interferon therapy, while 197 patients did not respond with any antibody development at all. In a replication sample comprising 865 patients diagnosed with MS, IFNβ-neutralizing antibody development was identified in 159 patients while no antibody titers at all were detected in 498 individuals of this cohort. This sums up to 301 patients developing neutralizing antibodies and 695 patients developing no antibodies againtst IFNβ in the combined sample of both patient collectives.

EDTA blood was collected after informed consent. DNA was extracted and quantified using standard protocols. Genotyping was performed on lllumina 370 CNV chips.

Statistical analyses were performed by comparing the allele frequencies of the respective SNP alleles in patients and controls using a χ² test with 1 degree of freedom implemented in PLINK V.1.0.2.

**Table 2**

| SNP | Effective sample size | OR | L95 | U95 | p |
|---|---|---|---|---|---|
| rs184452 | 739 | 0.644029851945149 | 0.510176899317201 | 0.813001236927048 | 0.0001891433 |
| rs2395175 | 931 | 0.492810973182889 | 0.3610228955804 | 0.672707072771734 | 9.053591e-06 |
| rs34369284 | 886 | 0.542444555843804 | 0.43400425707555 | 0.67797974643682 | 2.58821e-08 |
| rs9271300 | 875 | 0.575026012544628 | 0.463511117633314 | 0.713369976520298 | 2.708485e-07 |
| rs9272105 | 890 | 1.99158638589535 | 1.60397791396336 | 2.47286218716245 | 1.144748e-10 |
| rs2281389 | 931 | 1.75615542128377 | 1.32561409500712 | 2.32653068138039 | 4.815578e-05 |
| rs9496948 | 920 | 0.375837691624694 | 0.237257247977711 | 0.595362087564334 | 2.954203e-05 |
| rs300148 | 932 | 1.72349723038119 | 1.28050130270781 | 2.31974984863365 | 0.0001896450 |
| rs4607409 | 928 | 1.87955322063396 | 1.35445070990634 | 2.60823098497235 | 7.192383e-05 |
| rs16912375 | 932 | 0.507512671451784 | 0.341023021584311 | 0.755283647677281 | 0.0009479353 |
| rs4581054 | 931 | 1.99774291344906 | 1.34075392722219 | 2.97666608853764 | 0.0007696207 |
| rs4961252 | 924 | 1.59481046746418 | 1.30204647209148 | 1.9534021877482 | 5.426055e-06 |
| rs1434302 | 747 | 2.41723228070022 | 1.56193739095667 | 3.74087459118984 | 8.650364e-05 |
| rs2344827 | 920 | 1.65306890039523 | 1.25662271326697 | 2.17458809283304 | 0.0003513593 |
| rs1882545 | 754 | 0.694846167252398 | 0.552797093677493 | 0.873396770112225 | 0.001761174 |
| rs7183534 | 932 | 1.77404278249965 | 1.33686242694422 | 2.35418972865665 | 7.838401e-05 |
| rs3810481 | 911 | 0.607678633546897 | 0.468085701952258 | 0.78890109253345 | 0.0001960494 |

The above list inter alia contains those SNPs that are significant genome-wide, i.e. with a P-value of less than 5e-08, as generally agreed upon in the art. All analysis were done a generalized linear model, including adjustment for age, sex, the type of interferon and the subtype of multiple sclerosis. Building a classifier based on the larger list, it is possible (applying support vector methodology with a RBF kernel as implemented in DTREG 9.5) to predict the development of neutralizing antibodies or not correctly in 73.1 % of the patients (figure based on the 10-fold cross validation data set). This classifier contains the following SNPs, ranked based on their relative importance (max set to 100). In this regard, the term importance is defined as relative contribution to the calsssifier performance.

| SNP | Importance |
|---|---|
| rs7183534 | 100.000 |
| rs9496948 | 78.947 |
| rs34369284 | 73.684 |
| rs9272105 | 73.684 |
| rs9271300 | 42.105 |
| rs2344827 | 36.842 |
| rs2281389 | 26.316 |
| rs3810481 | 21.053 |
| rs1434302 | 15.789 |
| rs4607409 | 15.789 |
| rs2395175 | 10.526 |
| rs1882545 | 10.526 |
| rs300148 | 5.263 |

This corresponds to an area under the curve of 0.72 in the test data.

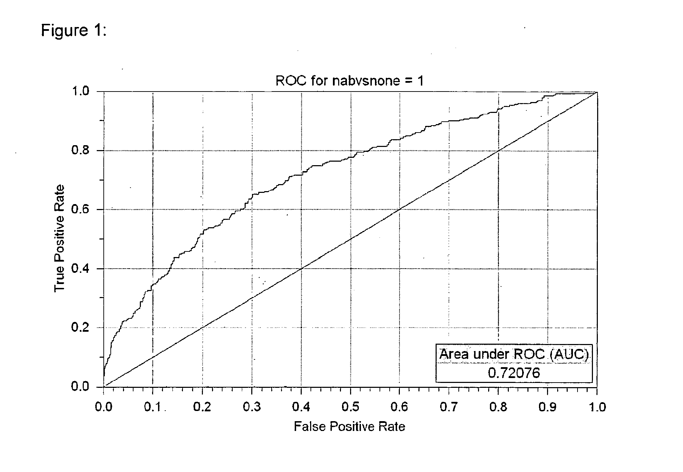

The illustration in Figure 1 shows the ROC curve of this classifier.

### Example 2: Polymorphisms can be used to predict the titer of antibodies to IFNβ in MS patients treated with interferon drugs

390 patients diagnosed with MS were selected according to their antibody response to interferon treatment. 193 patients responded with either binding or neutralizing AB development to interferon therapy, while 197 patients did not respond.
EDTA blood and serum was collected after informed consent. The study was approved by the local ethic committee. DNA was extracted using kits provided by Amersham or Quiagen. Amount of DNA was quantified by UV-absorption or quantitative PCR (RNAseP). Quality of the DNA was assessed by absorption at 260/280 and 260/230 PCR in selected samples.
Genotyping was performed on Illumina 370 CNV chips.

Statistical analyses were performed by comparing the allele frequencies of the respective SNP alleles in patients and controls using a χ² test with 1 degree of freedom implemented in PLINK V.1.0.2.

**Table 3**

| SNP | effective_sample_size | explained variance_(r²) | p-value |
|---|---|---|---|
| rs184452 | 919 | 0.001825176 | 0.1937360 |
| rs2395175 | 1164 | 0.009424346 | 0.0008500397 |
| rs34369284 | 1109 | 0.02637269 | 4.503145e-08 |
| rs9271300 | 1097 | 0.01788701 | 7.967568e-06 |
| rs9272105 | 1114 | 0.03288888 | 8.775828e-10 |
| rs2281389 | 1164 | 0.00837528 | 0.001668584 |
| rs9496948 | 1149 | 0.009850781 | 0.000705231 |
| rs300148 | 1165 | 0.007353611 | 0.003217493 |
| rs4607409 | 1161 | 0.01007255 | 0.0005723734 |
| rs16912375 | 1165 | 0.00727193 | 0.003393028 |
| rs4581054 | 1164 | 0.007372075 | 0.003192045 |
| rs4961252 | 1156 | 0.02542485 | 4.166917e-08 |
| rs1434302 | 929 | 0.01114245 | 0.001215214 |
| rs2344827 | 1152 | 0.001525953 | 0.1826997 |
| rs1882545 | 928 | 0.002735177 | 0.1098263 |
| rs7183534 | 1165 | 0.01141996 | 0.0002376076 |
| rs3810481 | 1140 | 0.01217193 | 0.0001745840 |

**Table 4: SNPs indicative for the development of neutralizing antibodies against IFNβ in MS patients**

| CHR | SNP | gene | position | minor_allele | major_allele | minor_allele_ frequency |
|---|---|---|---|---|---|---|
| 3 | rs184452 | PRICKLE2 | 64125246 | G | T | 0.4229947 |
| 6 | rs2395175 | | 32513004 | A | G | 0.1090067 |
| 6 | rs34369284 | | 32622122 | T | C | 0.3480565 |
| 6 | rs9271300 | | 32689560 | G | C | 0.4330357 |
| 6 | rs9272105 | | 32707977 | A | G | 0.4419525 |
| 6 | rs2281389 | | 33167774 | C | T | 0.1847643 |
| 6 | rs9496948 | UTRN | 1,45E+08 | C | T | 0.04688832 |
| 6 | rs300148 | | 1,66E+08 | C | T | 0.1597981 |
| 6 | rs4607409 | | 1,66E+08 | C | T | 0.1354430 |
| 8 | rs16912375 | | 85085177 | A | G | 0.06139613 |
| 8 | rs4581054 | | 85127338 | G | A | 0.06102694 |
| 8 | rs4961252 | | 1,42E+08 | G | A | 0.4067797 |
| 9 | rs1434302 | | 28965706 | T | G | 0.06402116 |
| 11 | rs2344827 | TRIM3 | 6442245 | G | A | 0.1556122 |
| 12 | rs1882545 | LAG3 | 6755671 | C | T | 0.3597884 |
| 15 | rs7183534 | | 24061503 | T | C | 0.1253154 |
| 20 | rs3810481 | PRIC285 | 61663889 | A | G | 0.1752577 |

**Table 5: SNPs indicative for the development of high antibody titers against IFNβ in MS patients**

| CHR | SNP | position | minor_allele | major_allele | minor_allele_frequency |
|---|---|---|---|---|---|
| 6 | rs2395175 | 32513004 | A | G | 0.1090067 |
| 6 | rs34369284 | 32622122 | T | C | 0.3480565 |
| 6 | rs9271300 | 32689560 | G | C | 0.4330357 |
| 6 | rs9272105 | 32707977 | A | G | 0.4419525 |
| 6 | rs9496948 | 144743809 | C | T | 0.04688832 |
| 6 | rs4607409 | 166395703 | C | T | 0.1354430 |
| 8 | rs4961252 | 142174126 | G | A | 0.4067797 |
| 9 | rs1434302 | 28965706 | T | G | 0.06402116 |
| 15 | rs7183534 | 24061503 | T | C | 0.1253154 |
| 20 | rs3810481 | 61663889 | A | G | 0.1752577 |

### References

Adams et al. (Ed.), "Automated DNA Sequencing and Analysis", Academic Press, 1994; Alphey, "DNA Sequencing: From Experimental Methods to Bioinformatics", Springer Verlag Publishing, 1997
Agrawal (Ed.), "Protocols for Oligonucleotides and Analogs: Synthesis and Properties (Methods in Molecular Biology, 20)", Humana Press, 1993
Amar et al., J. Clin. Microbiol. 40 (2002) 446-452
Ausubel, "Current Protocols in Molecular Biology", Green Publishing Associates and Wiley Interscience, N.Y. (1989)
Baranzini et al. (2005) PLoS Biol 3: 166-176
Barany (1991)
Barcellos et al. (2004) Ann Neurol 55:793-800
Barnes et al., Nucleic Acids Res. 33 (2005) 5914-5923
Barton et al., (2004) Brain 127:1717-1722
Boz et al. (2007) Mult Scler 13: 1127-1137
Burczak and Mardis (Ed.), "Polymorphism Detection & Analysis Techniques", Eaton Pub Co, 2000
Chen and Janes (Ed.), "PCR Cloning Protocols: From Molecular Cloning to Genetic", 2nd edition, Humana Press, 2002
Cotton et al. (Ed.), "Mutation Detection: A Practical Approach", Irl Press, 1998;
Ebers et al. (1986) N Engl J Med 315: 1638-1642
Egholm, M., Buchardt, O., Christensen, L., Behrens, C., Freier, S.M., Driver, D.A., Berg, R.H., Kim, S.K., Norden, B., Nielsen, P.E., PNA hybridizes to complementary oligonucleotides obeying the Watson-Crick hydrogen-bonding rules: Nature. 1993 Oct 7;365(6446):566-8
Fan et al., Biotechniques 39 (2005) 583-588
Farrell et al. (2008) Mult Scler 14: 212-218
Flavell et al., Nucleic Acids Res. 31 (2003) e115
Gneiss et al. (2006) J. Neuroimmunol 174: 174-179
Green et al (2001) J Neuroimmunol 116:116-124
Gregory et al. (2007) Nat Genet 39: 1083-1091
Gunderson et al., Nature Genetics 37 (2005) 549-554Francis et al. (2005) Neurology 65: 48-55
Haque et al., Diagn. Mol. Pathol. 7 (1998) 248-252
Hartung et al. (2004) J Neurol 251: v12-v29
He et al. (1998) J Neuroimmunol 86:13-19; Hultqvist et al. (2004) Proc Natl Acad Sci USA 101:12646-12651
Higgins and Hames (Eds.) "Nucleic acid hybridization, a practical approach" IRL Press Oxford, Washington DC, (1985)
Hsia et al., Theor. Appl. Genet. 111 (2005) 218-225
Innis et al. (Ed.), "PCR Applications: Protocols for Functional Genomics", Academic Press, 1999
ltakura et al., Ann Rev Biochem (1984), 53; 323-356
Kappos et al. (2005) Neurology 65: 40-47
Kakavas et al., J. Clin. Lab. Anal. 20 (2006) 1-7
Kesselring Ed. "Epidemiologie." 3 ed. Psychiatrie, Neurologie, Klinische Psychologie. Stuttgart: Kohlhammer, 70-76, 1997
Killestein et al. (2002) J Neuroimmunol 133: 217-224
Kurtzke (1983) Neurology 33:1444-1452
Landegren (1993)
Lassmann et al. (2001) Trends Mol Med 7: 115-121
Leyva et al. (2005) J Neuroimmunol 163:165-171
Li et al., Electrophoresis (1999), 20; 6, 1258-1265
Lohara et al., Nucleic Acids Res. 30 (2002) e87
Lucchinetti et al. (2000) Ann Neurol 47:707-717
Lundmark et al. (2007) Nat Genet 39: 1108-1113
McDonnell et al. (1999) Mult Scler 5:105-109
Meng et al., J. Clin. Endocrinol. Metab. 90 (2005) 3419-3422
Newton et al., Nucleic Acids Res. 17 (1989) 2503-2516
Nielsen, P.E., Egholm, M., Berg, R.H., Buchardt, O., Sequence-selective recognition of DNA by strand displacement with a thymine-substituted polyamide. Science. 1991 Dec 6;254(5037):1497-500
Nollau et al, Clin. Chem. 43 (1997), 1114-1128
Palais et al., Anal. Biochem. 346 (2005) 167-175
Petereit et al. (2002) Mult Scler 8: 492-494
Petersen et al. (2006) Mult Scler 12: 247-252
Petersen et al., Hum. Mutat. 20 (2002) 253-259
Pissardet al., Clin. Chem. 48 (2002) 769-772
Prineas "The Neuropathology of Multiple Sclerosis." Ed. J. C. Koetsier. Amsterdam: Elsevier Science Publishers, 213-257, 1985; Lassmann (1998) Mult Scler 4: 93-98
Ramagopalan et al. (2007) N Engl J Med 357: 2199-2200;
Ramon et al., J. Transl. Med. 1 (2003) 9;
Rio et al. (2002) Ann Neurol 52: 400-406
Rudick et al. (2004) Ann Neurol 56: 548-55
Sadovnick et al. (1988) Am J Med Genet 29: 533-541
Sambrook. Russell "Molecular Cloning, A Laboratory Manual", Cold Spring Harbor Laboratory, N.Y. (2001)
Sanger et al. (1977), DNA sequencing with chain-terminating inhibitors. Proc Natl Acad Sci U S A 74:5463-7
Shen et al., Mutat. Res.-Fund. Mol. M. 573 (2005) 70-82
Sorensen et al. (2005) Neurology 65: 33-39
Steemers, F.J., Gunderson, K.L., Illumina, Inc. Pharmacogenomics. 2005 Oct;6(7):777-82
Steemers et al., Nature Meth. 3 (2006) 31-33
Stürzebecher et al. (2003) Brain 126: 1419-1429
Taylor (Ed.), "Laboratory Methods for the Detection of Mutations and Polymorphisms in DNA", CRC Press, 1997
Taylor and Day (Ed.) "Guide to Mutation Detection", Wiley, 2005
The International Multiple Sclerosis Genetics Consortium (2007) N Engl J Med 357: 851-862
Todd et al., J. Oral Maxil. Surg. 59 (2001) 660-667
Tomassini et al. (2006) J Neurol 253: 287-293
Tost and Gut, Clin. Biochem. 35 (2005) 335-350
Villoslada et al. (2002) J Neuroimmunol 130:194-201
Vyshkina et al (2004) Mult Scler 10:614-617 7
Wandinger et al. (2003) Lancet 361: 2036-2043
Waubant et al. (2003) Neurology 61: 184-189
Weber et al. (2008) Genes lmmun. 9(3): 259-63
Weinshenker et al. (2001) J Neuroimmunol 120:138-145
Wiedmann et al. (1994)
Youil et al., Proc. Natl. Acad. Sci. U.S.A. 92 (1995) 87-91

## Claims

1. A method of diagnosing a predisposition of a multiple sclerosis patient to become less susceptible or resistant to treatment with interferon β, said method comprising determining in a sample obtained from said patient the presence of one or more SNP(s) selected from the group consisting of SNPs defined by the sequences of SEQ ID NOs 1, 2, 3, 4, 5, 6, 7, 8, 9, 10, 11, 12, 13, 14, 15, 16 and 17,
wherein the presence of one or more of said SNP(s) in said sample is indicative for said predisposition.

2. A method of diagnosing a predisposition of a multiple sclerosis patient for developing neutralizing antibodies against interferon-β, said method comprising determining in a sample obtained from said patient the presence of one or more SNP(s) selected from the group consisting of SNPs defined by the sequences of
SEQ ID NOs 1, 2, 3, 4, 5, 6, 7, 8, 9, 10, 11, 12, 13, 14, 15, 16 and 17,
wherein the presence of one or more of said SNP(s) in said sample is indicative for said predisposition.

3. A method of diagnosing a predisposition of a multiple sclerosis patient for developing high antibody titers against interferon-β, said method comprising assessing in a sample obtained from said patient the presence of one or more SNP(s) selected from a subgroup consisting of SNPs defined by the sequences of
SEQ ID NOs 3, 5, 6, 7, 8, 9, 12, 13, 16 and 17;
wherein the presence of one or more of said SNP(s) selected from said subgroup in said sample is indicative for both said predispositions.

4. The method of claim 2, further comprising diagnosing a predisposition of said patient for developing high antibody titers against interferon-β, wherein the one or more SNP(s) are selected from a subgroup consisting of SNPs defined by the sequences of SEQ ID NOs. 3, 5, 6, 7, 8, 9, 12, 13, 16 and 17, wherein the presence of one or more of said SNP(s) from said subgroup in said sample is indicative for both said predispositions.

5. The method of claims any of claims 1 to 4, wherein said one or more SNP(s) are selected from the group consisting of SNPs defined by the sequences of SEQ ID NOs 5, 8 and 12.

6. The method of any one of claims 1 to 5, wherein the presence of at least two SNPs selected from (each of) said group(s) is determined.

7. The method of any one of claims 1 to 6, wherein said determining is effected by any one of
(a) an assay based on physical separation of nucleic acid strands,
(b) ligase chain reaction assay,
(c) cleavage and digestion assay,
(d) sequencing assay,
(e) nucleic amplification assay, or
(f) hybridization assay.

8. The method of claim 7, wherein said nucleic acid amplification assay is a PCR assay making use of one or more forward primer(s) and one or more reverse primer(s), wherein at least one primer is identical or complementary to a region of a sequence of any one of SEQ ID NOs 1, 2, 3, 4, 5, 6, 7, 8, 9, 10, 11, 12, 13, 14, 15, 16 and 17 comprising position 200 of SEQ ID NOs 1 and 6, position 201 of SEQ ID NOs 2, 3, 4, 5, 7, 8, 9, 10, 11, 12, 13, 14, 15, 16 and 17, and position 206 of SEQ ID NO 15.

9. The method of claim 7, wherein the nucleic acid amplification assay is a primer extension assay making use of one or more primer(s), wherein at least one primer is identical or complementary to a region of a sequence of any one of SEQ ID NOs 1, 2, 3, 4, 5, 6, 7, 8, 9, 10, 11, 12, 13, 14, 15, 16 and 17 immediately upstream of position 200 of SEQ ID NOs 1 and 6, position 201 of SEQ ID NOs 2, 3, 4, 5, 7, 8, 9, 10, 11, 12, 13, 14, 15, 16 and 17, and position 206 of SEQ ID NO 15.

10. The method of claim 7, wherein the hybridization assay makes use of one or more nucleic acid molecule(s) as probe(s), wherein at least one probe is identical or complementary to a region of a sequence of any one of SEQ ID NOs: 1, 2, 3, 4, 5, 6, 7, 8, 9, 10, 11, 12, 13, 14, 15, 16 and 17 comprising position 200 of SEQ ID NOs 1 and 6, position 201 of SEQ ID NOs 2, 3, 4, 5, 7, 8, 9, 10, 11, 12, 13, 14, 15, 16 and 17, and position 206 of SEQ ID NO 15.

11. A solid support comprising one or more of the primer(s) of claim 8 or 9, and/or one or more of the probe(s) of claim 10.

12. A diagnostic composition for diagnosing a predisposition of a multiple sclerosis patient to become less susceptible or resistant to treatment with interferon β, said composition comprising one or more of the primer(s) of claim 8 or 9, one or more of the probe(s) of claim 10, and/or one or more of the solid support(s) of claim 11.

13. The method of any one of claims 1 to 10 or the diagnostic composition of claim 12, wherein said patient has a Caucasian genetic background.

14. A kit comprising the solid support of claim 11 or the diagnostic composition of claim 12 in one or more containers and, optionally, instructions for using said kit or diagnostic composition.
